# EUROPEAN PATENT SPECIFICATION

(11) **EP 1 730 531 B1**
(45) Date of publication and mention of the grant of the patent: **15.01.2014**
(21) Application number: 05718906.0
(22) Date of filing: 24.03.2005
(51) Int. Cl.: G01N 33/543, G01N 27/60

(54) **ELECTRODE**
ELEKTRODE
ELECTRODE

(30) Priority: 24.03.2004 US 555670 P
(43) Date of publication of application: 13.12.2006
(73) Proprietor: Technion Research and Development Foundation, Limited, Haifa 3200004 (IL)
(72) Inventor: SIVAN, Uri, 34602 Haifa (IL); REITER, Yoram, 34987 Haifa (IL); ARTZY-SCHNIRMAN, Arbel, 34464 Haifa (IL)
(74) Representative: Dennemeyer & Associates S.A.
(86) International application number: PCT/IL2005/000333
(87) International publication number: WO 2005/090981

(56) References cited:
- WO-A-01/02858
- WO-A-99/66322
- WO-A-03/021268
- US-A- 5 716 854
- US-A- 5 728 532
- US-A1- 2002 028 440
- US-B1- 6 325 904
- US-B1- 6 342 347
- ESTRELA P ET AL: "Electrical detection of biomolecular interactions with metal-insulator-semiconductor diodes" BIOSENSORS & BIOELECTRONICS, ELSEVIER SCIENCE PUBLISHERS, BARKING, GB, vol. 20, no. 8, 15 February 2005 (2005-02-15), pages 1580-1586, XP004697377 ISSN: 0956-5663
- CARUSO F ET AL: "DNA binding and hybridization on gold and derivatized surfaces" SENSORS AND ACTUATORS B, ELSEVIER SEQUOIA S.A., LAUSANNE, CH, vol. 41, no. 1-3, 30 June 1997 (1997-06-30), pages 189-197, XP004091223 ISSN: 0925-4005
- WHALEY ET AL: "Selection of peptides with semi-conductor binding specifically for directed nanocrystal assembly" NATURE, vol. 405, 8 June 2000 (2000-06-08), pages 665-668, XP002342143 cited in the application

## Description

The present invention concerns an electrode according to claim 1, an array comprising a plurality of addressable locations according to claim 7, a kit for typing ligands according to claim 9, a method according to claim 10, a device for controllable delivery of a drug molecule to a tissue according to claim 13.

The present disclosure relates to an artificial receptor capable of binding specific biological moieties, and more particularly, to methods of using same for typing ligands, determining binding domains in proteins, targeted delivery and release of drug molecules, and gaining electrical control over biological processes.

Electrostatic interactions underlie the basis of various biological processes involving the recognition and binding of macromolecules such as DNA, RNA, proteins and carbohydrates to each other. For example, alien macromolecules are identified through molecular recognition between an antibody molecule and the intruding molecule, generally denoted antigen. Likewise, ligands such as hormones bind to their cellular receptors and thus activate cellular responses.

Characterization of the domain structures involved in protein-protein interactions such as those between ligands and receptors or antibodies and antigens is crucial for gaining control over such biological processes. Such a characterization can be performed using site directed mutagenesis, in which targeted mutations are introduced into DNA sequences encoding specific proteins (e.g., a receptor) and the effect of the mutation is tested *in vitro* following the expression of the mutated DNA in suitable cells in the presence of a test molecule (e.g., a labeled ligand). Another approach for characterizing binding domain in a protein is crystallography of a purified protein in the presence of a labeled ligand. Such experiments often results in determination of the amino acids involved in binding the ligand. However, while the first approach is limited by the specific mutations introduced, the latter approach is relatively expensive due to the need of substantial purification steps of the protein of interest.

Most drug molecules are administered using oral or intravenous administration which often result in various unwanted side effects. Such effects result from the interaction of the drug molecule with tissues or organs not intended to be treated by the drug. To overcome such limitations, various targeted drug delivery approaches were developed. These include, viral infection, temperature-sensitive liposome formulations (Viglianti BL, et al., Magn Reson Med. 2004, 51: 1153-62), magnetoliposomes (Kullberg M. et al., Med Hypotheses. 2005, 64: 468-70), ultrasound-mediated microbubbles (Tsutsui JM, et al., Cardiovasc Ultrasound. 2004, 2: 23) and the like.
US-B1-6 342 347 (Bauer Alan Joseph) (2002-01-29) teaches a sensor for analyte detection which makes changes in electrostatic fields associated with macromolecular binding agents during their interaction with analytes.
WO 99/66322 A (Biosensor Systems Design)(1999-12-23) teaches a sensor for detecting analytes of interest by measuring changes in induced electromotive force, currently etc during analyte exposure to the sensor.
US-A-5 716 854 (Loefaas et al) (1998-02-10) teaches an assay method comprising the step of binding a second substance to a first substance immobilized to a solid phase circuit, the first substance being either a ligand or a species bound directly or indirectly to the ligand.
US 2002/028440 A1 (Willner Itamar et al) (2002-03-07) teaches binding between two members of a recognition pair, for example antigen-antibody, is determined by utilizing a probe comprising a piezoelectric crystal with electrodes on two opposite faces of the crystal.
US-B1-6 325 904 (Peeters John P) (2001-12-04) teaches an array of electrodes at the atomic or nano scale built on a chip. The spatial distribution, height, width and electrochemical composition of the nano-electrodes are varied such that protein specific electronic receptors are built directly on the chip without the use of specific binding agents or molecules.
US-A-5 728 532 (Ackley et al) (1998-03-17) teaches selectively attracting and inhibiting attraction of a predetermined molecule to a site in a molecular detection device using two electrodes proximate to the site. The electrodes selectively generate attractive and repulsive forces.
WO 03/021268 A (HRL Laboratories, (2003-03-13) teaches a concentrator for detecting biological or chemical materials in an environment. An engineered superlattice structure has alternate layers of binary and ternary Group III - Group V, or Group IV - Group IV semiconductor materials. The structure is exposed to the environment and activated optically or electrically.
WO 01/02858 A (Institute of Molecular Agrobiology) (2001-01-11) teaches a quantitative method for detection of viral or bacterial antibodies using an apparatus that includes a piezoelectric crystal, an oscillation circuit and a universal counter. BIOSENSORS & BIOELECTRONICS, vol. 20, no. 8,15 February 2005 (2005-02-15), pages 1580-1586 Estrela et al: "Electrical detection of biomolecular interactions with metal-insulator-semiconductor diodes", teaches label free detection of DNA hybridization using a metal insulator semiconductor (MIS) diode or capacitor. SENSORS AND ACTUATORS vol. 41, no. 1-3,30 June 1997 (1997-0630), pages 189-197, Caruso F et al: "DNA binding and hybridization on gold and derivatized surfaces", teaches the binding of various single and double stranded DNAs onto gold and derivativized gold surfaces and their hybridization with complementary DNA species.

There is thus a widely recognized need for, and it would be highly advantageous to have, methods of gaining control over biological processes, characterizing domain structures for protein-protein interactions and efficient targeted drug delivery devoid of the above limitations.

The electrode of the present invention is defined in claim 1, the array comprising a plurality of addressable locations of the present invention is defined claim 7, the kit for typing ligands of the present invention is defined claim 9, the method of the present invention is defined claim 10, the device for controllable delivery of a drug molecule to a tissue of the present invention is defined claim 13.

According to still another aspect of the present disclosure there is provided an array comprising a plurality of addressable locations each including an artificial receptor configured capable of a unique surface electrical property enabling the artificial receptor to specifically bind a ligand.

According to an additional aspect of the present disclosure there is provided a kit for typing ligands comprising an artificial receptor configured capable of a unique surface electrical property enabling to specifically bind a ligand and reagents for qualifying binding of the ligands to the plurality of artificial receptors.

According to yet an additional aspect of the present disclosure there is provided a method of identifying a small molecule capable of mimicking a binding function of a ligand, the method comprising: (a) exposing the antibody to at least one electrode configured capable of a unique surface electrical property enabling a specific binding of the ligand thereto, thereby identifying at least one electrode capable of specifically binding the ligand; and (b) identifying a small molecule of a plurality of small molecules capable of binding the at least one electrode being identified as capable of specifically binding the ligand, the small molecule being capable of mimicking the binding function of the ligand.

According to still an additional aspect of the present disclosure there is provided a method of isolating a specific ligand from a mixed population of ligands, the method comprising exposing the mixed population of ligands to at least one electrode configured capable of a unique surface electrical property enabling a specific binding of a ligand thereto, thereby isolating the specific ligand from the mixed population of ligands.

According to a further aspect of the present disclosure there is provided a device for controllable delivery of a drug molecule to a tissue comprising a device body including at least one electrode configured capable of a unique surface electrical property enabling a specific binding of a ligand thereto; the ligand being attached to the drug, the unique surface electrical property capable of being modified by a switching unit to control a binding or a release of the ligand and thereby controllably deliver the drug molecule to the tissue.

According to further features in preferred embodiments of the disclosure described below, the surface further comprises zero dimension, one dimension, two dimensions and/or three dimensions.

According to still further features the target biological moiety is not a peptide.

According to still further features the surface further comprises at least one substance attached to the surface, the at least one substance being capable of modifying hydrophobic property, charged state, hydrophilic property and/or said electrical property of said surface.

According to still further features the at least one substance capable of modifying hydrophobic interaction, hydrophilic interaction, hydrogen bonding and van der Wallc interaction of said surface with said target biological moiety.

According to still further features the at least one substance is an organic substance.

According to still further features the organic substance is selected from the group consisting hydroquinone and rotaxane.

According to still further features the at least one substance is a biological substance.

According to still further features the biological substance is a peptide, a protein, a lipid, and/or a nucleic acid.

According to still further features the at least one substance is a Ferroelectric substance.

According to still further features the Ferroelectric substance is PLZT, and/or BaTiO₃.

According to still further features the at least one substance is a Pyroelectric substance.

According to still further features the Pyroelectric substance is Li-Ta-O₃, polyvinylidene fluoride (PVDF), and/or lead Titanate (PT).

According to still further features the at least one substance is a Piezoelectric substance.

According to still further features the Piezoelectric substance is PZT.

According to still further features the surface comprises a plurality of regions over the extent, each region having a predetermined electrostatic field strength.

According to still further features each of the regions comprises a respective material selected for electrostatic properties thereof.

According to still further features the respective material is selected from the group consisting of a ceramic and a semiconductor.

According to still further features the respective material is selected from the group consisting of a Ferroelectric material, a Pyroelectric material, and a Piezoelectric material.

According to still further features the Ferroelectric material is PLZT and/or BaTiO₃.

According to still further features the Pyroelectric material is Li-Ta-O3, polyvinylidene fluoride (PVDF), and/or lead Titanate (PT).

According to still further features the Piezoelectric material is PZT.

According to still further features the regions are in the nanometer or Angstrom orders of magnitude.

According to still further features the regions are in the order of magnitude of five to ten lattice constants.

According to still further features variable electrical fields are applied to the regions.

According to still further features the regions comprise crystals or polycrystals placed in between electrodes.

According to still further features the crystal comprises a high dielectric constant ceramic.

According to still further features the high dielectric constant ceramic comprises PLZT.

According to still further features the artificial receptor further comprises a laminate including a plurality of independently electrifiable layers, the surface being formed from a cross-section of the laminate such that the independently electrifiable layers form respective ones of the regions.

According to still further features the artificial receptor further comprises a selector for selecting a voltage level for each of the independently electrifiable layers, thereby to allow the electrical field to be varied to provide specific binding to different targeted biological moieties.

According to still further features the surface comprises an electronically controllable hydrophobic coating, thereby to allow controllable hydrophobic properties per independently electrifiable layer.

According to still further features the artificial receptor further comprises insulating layers between the independently conductive layers.

According to still further features the artificial receptor further comprises a covering layer located over the laminate.

According to still further features the covering layer comprises glass.

According to still further features the covering layer comprises cavitation.

According to still further features the surface comprises switchable wiring, the wiring being switchable to provide the unique electrical properties.

According to still further features the wiring is variably switchable, thereby to provide the specific binding to different target biological moieties as desired.

According to still further features the plurality of layers comprise alternately insulating layers and conductive layers over at least part of the surface.

According to still further features the artificial receptor further comprises a switching unit for switching the layers such as to configure an electrical field about the surface to provide specific binding for a target biological moiety.

According to still further features the artificial receptor further comprises a covering layer over the surface.

According to still further features the covering layer comprises electrical insulation.

According to still further features the covering layer comprises cavitation.

According to still further features the cavitation is substantially at the nanometer or Angstrom scale.

According to still further features the artificial receptor plurality of layers have a transverse direction and a longitudinal direction at the surface and wherein the surface has a transverse direction and a longitudinal direction and wherein the layers are aligned about the surface such that the layer transverse direction lies along the surface longitudinal direction.

According to still further features the widths of the layers in the layer transverse direction are substantially at the nanometer or Angstrom scale.

According to still further features the artificial receptor further comprises a switching control for switching the conductive tracks such as to reconfigure an electrical field about the surface to provide specific binding for a target biological moiety.

According to still further features the widths of the conductive tracks are substantially in the nanometer or Angstrom order of magnitude.

According to still further features the artificial receptor includes at least one electrode selected of a size, shape or makeup enabling the unique surface electrical property.

According to still further features the least one electrode comprises a non-biological material.

According to still further features the at least one electrode is selected of a size or shape enabling binding of a biological moiety thereto.

According to still further features the at least one electrode is a plurality of electrodes whereas a combined surface electrical property of the plurality of electrodes is capable of binding a specific biological moiety.

According to still further features the at least one electrode includes a non-biological crystal structure having the unique surface electrical property.

According to still further features the at least one electrode includes a crystal structure having the unique surface electrical property.

According to still further features the at least one electrode is a semi-conductive electrode.

According to still further features the at least one electrode is composed of conductive and non-conductive layers.

According to still further features the array is constructed such that the unique surface electrical property of the electrode is modifiable.

According to still further features the size of each of the plurality of electrodes is in a nanometer range.

According to still further features the distance between each of the plurality of electrodes is smaller than 50 nanometer.

According to still further features the distance between each of the plurality of electrodes is smaller than 20 nanometer.

According to still further features the biological moiety is selected from the group consisting of a protein, a peptide, a DNA, an RNA, a carbohydrate and a lipid.

According to still further features the at least one electrode is a plurality of electrodes whereas a combined surface electrical property of the plurality of electrodes is capable of binding the ligand thereto.

According to still further features each of the plurality of electrodes is selected of a size or shape enabling binding of the ligand thereto.

According to still further features the combined surface electrical property of the plurality of electrodes is capable of binding the ligand thereto.

According to still further features the plurality of electrodes includes a non-biological crystal structure having the unique surface electrical property.

According to still further features each of the plurality of electrodes includes a crystal structure having the unique surface electrical property.

According to still further features each of the plurality of electrodes is a semi-conductive electrode.

According to still further features each of the plurality of electrodes is composed of conductive and non-conductive layers.

According to still further features each of the plurality of electrodes is constructed such that the unique surface electrical property of each electrode is modifiable.

According to still further features the size of each of the plurality of electrodes is in a nanometer range.

According to still further features the ligand is selected from the group consisting of a protein, a peptide, a DNA, an RNA, a carbohydrate and a lipid.

According to still further features the at least one electrode is selected of a size or shape enabling binding of the ligand thereto.

According to still further features the at least one electrode is constricted such that the unique surface electrical property is modifiable.

According to still further features the size of the at least one electrode is in a nanometer range.

According to still further features the ligand is selected from a phage display antibody library.

According to still further features the small molecule is a peptide and/or a peptide mimetic.

According to still further features the ligand is a biological moiety selected from the group consisting of a protein, a peptide, a DNA, an RNA, a carbohydrate and a lipid.

According to still further features modifying is effected using a remote switching unit.

According to still further features the method further comprises administering the drug molecule to the subject.

According to still further features administering is effected by intravenous administration and/or oral administration.

According to still further features the semiconductor nanocrystals are remotely electrifiable via incident radiation.

According to still further features the artificial receptor further comprises a substance storage and release mechanism associated with the surface, such that a given change in the electric field is operable to affect the storage and release mechanism to effect release of a substance stored therein.

The present invention may successfully address the shortcomings of the presently known configurations by providing an artificial receptor capable of specifically binding biological moieties.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

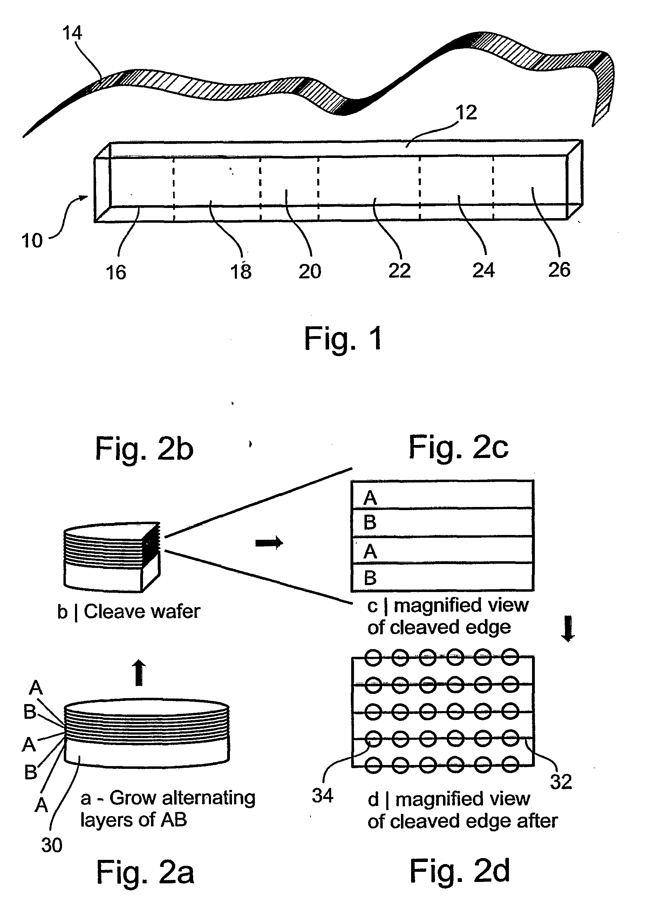

FIG. 1 is a schematic illustration of a specific artificial receptor device according to a first preferred embodiment of the present invention; a device 10 is set up with a surface 12 and an electric field about that surface. An isoelectric contour 14 illustrates a possible shape for the electrostatic potential. The surface includes a plurality of regions (16 - 26), each having a predetermined electrostatic field strength determined, e.g. by the voltage applied to it;
FIGs. 2a-d are schematic illustrations showing the construction of a programmable artificial receptor device according to a second preferred embodiment of the present invention. Figure 2a illustrates growing of alternating layers of conducting (A) and insulating (B) materials, e.g., GaAs/AlGaAs or metal/metal oxide. Figure 2b illustrates cleaving of the wafer. Figure 2c illustrates the cleaved surface comprises alternating strips with atomically sharp interfaces. Figure 2d shows the cleaved edge coated with glass and holes being etched in the glass, just on top of the A/B interfaces;

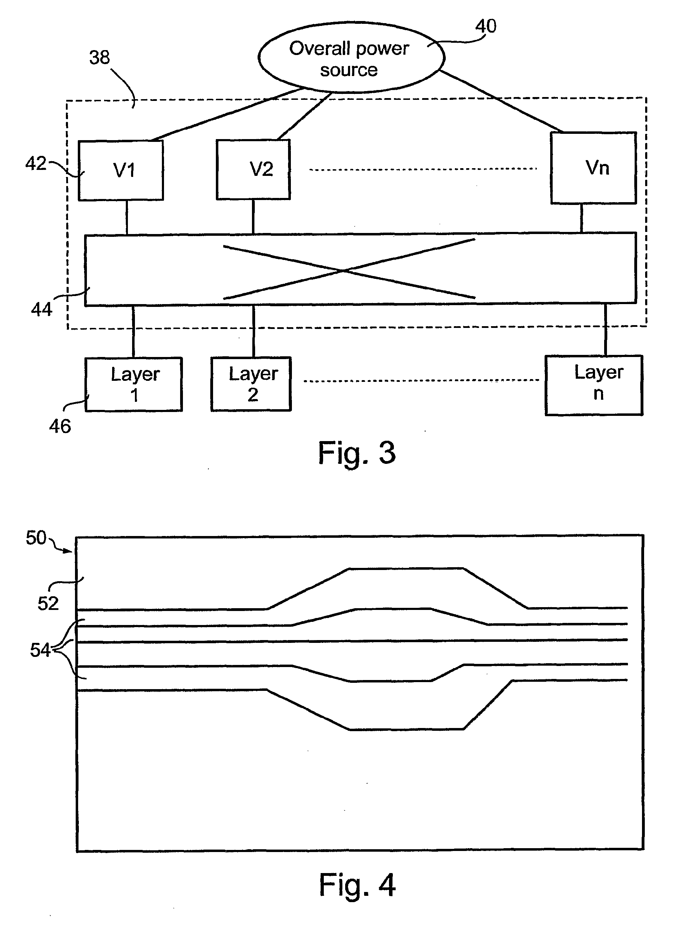

FIG. 3 is a simplified diagram showing a preferred switching arrangement for switching the layers of the device of Figures 2a-d to provide different voltage levels at the layers and a variable overall electrical field at the surface. Device or selector 38 allows selecting the voltage levels for each of the independently electrifiable layers. Power source 40 (may be a battery or a main fed power supply) supplies a series of voltage regulated power sources 42.1...42.n, each set at different voltage levels. A switching matrix 44 then connects any one of the layers 46.1...46.n to any one of the regulated power sources;
FIG. 4 is a simplified diagram showing a programmable artificial receptor device according to a third preferred embodiment of the present invention. Device 50 comprises a conventional semiconductor wafer surface 52 on which are patterned conductive tracks 54 using conventional semiconductor manufacturing techniques. The conductive tracks are switched using transistors in the conventional manner;

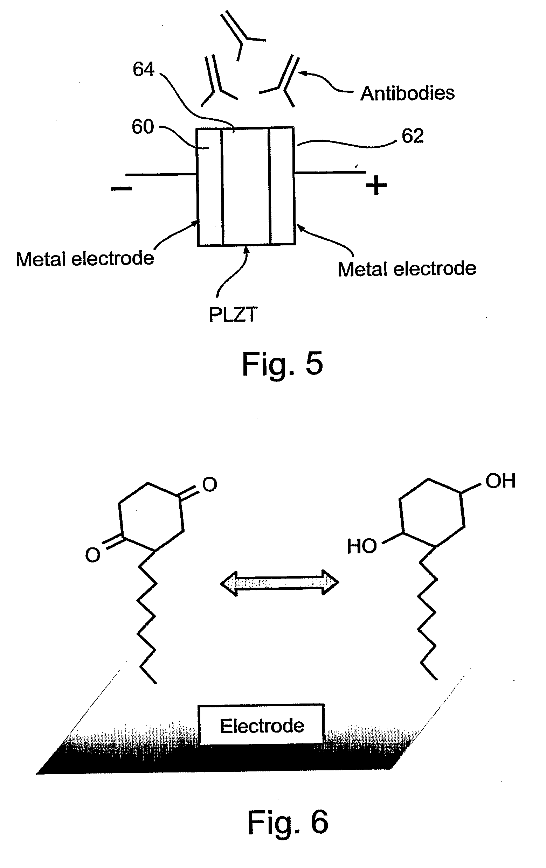

FIG. 5 is a schematic illustration of an artificial receptor based on a PLZT ferroelectric ceramics. The ceramics (64) is held between to two electrodes (60 and 62) and the application of field by the electrodes on the ceramics changes its unit cell structure. Antibodies selected against either unit cell structure bind to one configuration and do not bind the other configuration;
FIG. 6 is a schematic illustration depicting the effect of application of an electric field on the molecular structure of Hydroquinone. Under a certain electric field the hydroquinone molecule looses two hydrogen (H) molecules and a double bond (=) with oxygen (O) is formed;

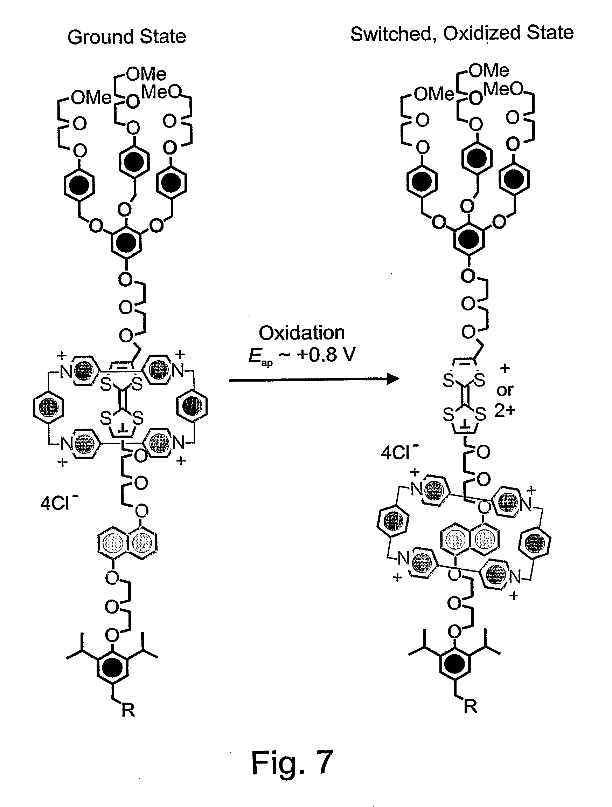

FIG. 7 is a schematic illustration depicting the effect of application of an electric field on the molecular structure of Rotaxane; Under a certain electric field the mobile molecular ring translocates into a different position in the molecule.

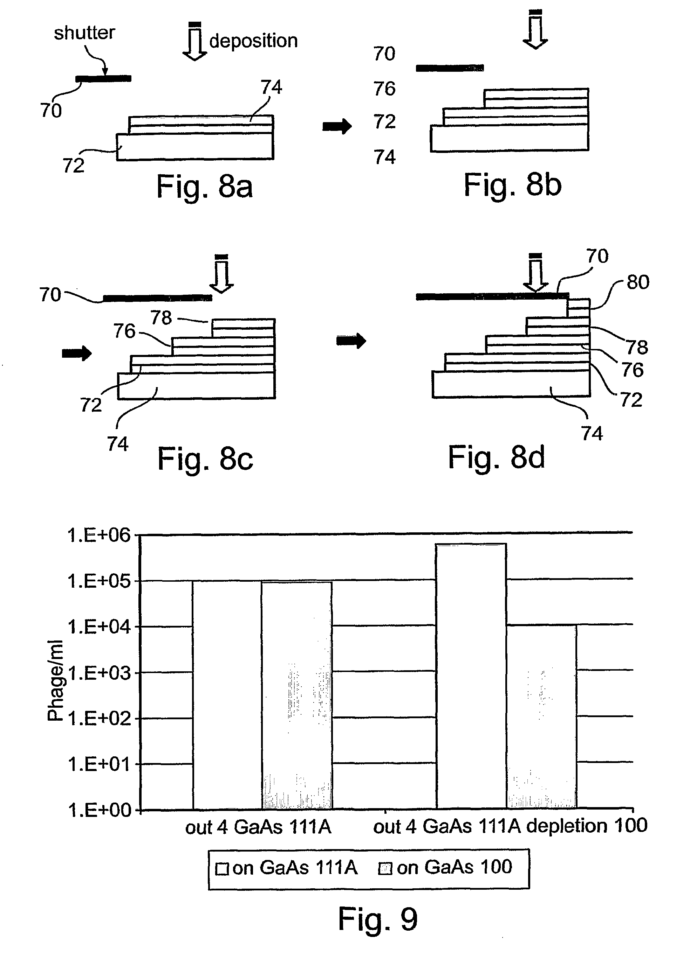

FIGs. 8a-d are a sequence of schematic illustrations showing successive stages in the manufacture of electrode layers to enable each layer to have a separate electrical contact. Note the receding shutter whose purpose is to leave exposed conducting segments of the conducting layers for later electrical contact;
FIG. 9 is a bar graph depicting the number of phages following each panning step and the effect of the depletion step prior to panning on the specificity of binding of phages to GaAs (111A) or GaAs (100) surfaces. Depletion was performed at the second and third rounds of panning by exposing the phage display library to the GaAs (100) surface prior to exposing the same phages to the GaAs (111A) surface. The number of phages bound to each surface following the fourth round of panning is presented. Note the significant increase in binding efficacy (by almost two orders of magnitudes) towards the GaAs (111) surface as compared with the GaAs (100) surface of phages selected following two depletion cycles.

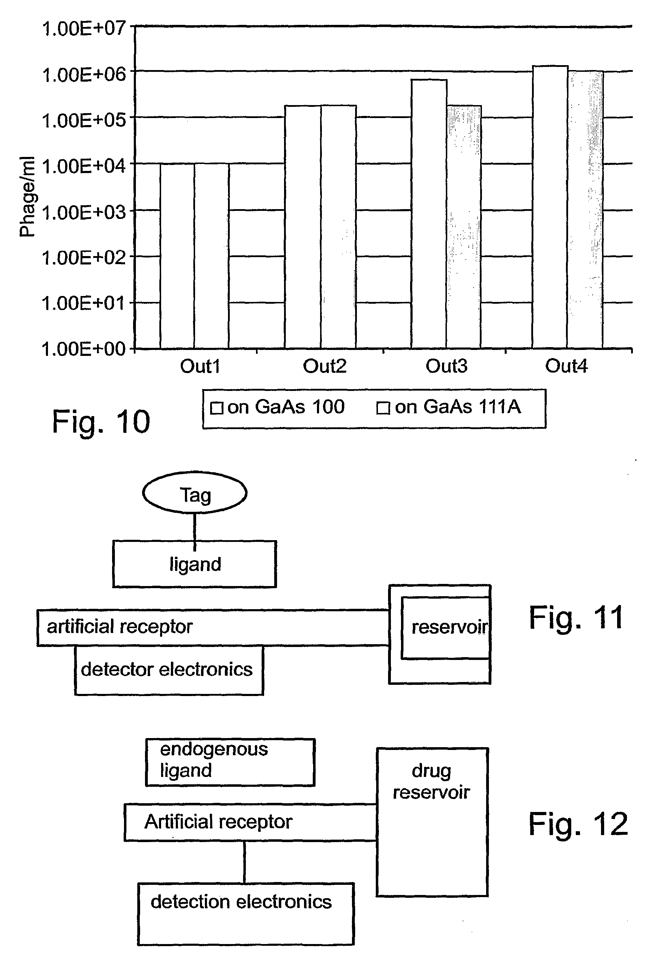

FIG. 10 is a bar graph depicting the enrichment of peptide binders to GaAs (100) and GaAs (111A) surfaces following each panning round.
FIG. 11 is a schematic illustration depicting a controlled drug release. A quantity of the drug to be released is held in a reservoir, and in the meantime a molecule for which the artificial receptor has an affinity is released into the bloodstream. The molecule that is released has a magnetic particle attached thereto, thus enabling the attachment of the particle to be sensed at the device. The molecule with the magnetic particle reaches the artificial receptor and binds thereto. The magnetic particle is detected via its magnetic field. Detection of the magnetic particle triggers release of the drug. The reservoirs can be placed with the devices deep inside the body at the points where drug administration is required. The particles can then be systematically administered to control release of the drug at the device. The particles can be used to ensure that a given quantity of the drug is released using timing based say on the half-life of the drug within the body.
FIG. 12 a schematic illustration depicting a binding of an endogenous ligand to the artificial receptor. Binding of the ligand affects the electric field of the device temporarily and may cause a temporary signal spike which may be detected following suitable noise reduction. The ligand may be selected to be representative of biological activity that it is desired to monitor. For example the ligand may be an antibody, and the presence or level of too many of the antibodies may indicate a certain condition. The condition may be treatable with a given drug which can be part of a controllable release feature as before.

### DESCRIPTION OF THE PREFERRED EMBODIMENTS

The present invention relates to an artificial receptor capable of binding specific biological moieties, and more particularly, to methods of using same for typing ligands, determining binding domains in proteins, targeted delivery of drug molecules, electronic capture and release of drugs, and electronic triggering and suppression of biological reactions such as gene expression.

The principles and operation of an artificial receptor device according to the present invention may be better understood with reference to the drawings and accompanying descriptions.

Specific binding of biological moieties (*i.e.,* biomolecules) to artificial substrates has been previously shown. For example, in recent years, following the work by S. Brown (S. Brown, 1997, Nature Biotechnolog 15: 269-272) several authors have shown that the remarkable molecular recognition capabilities of peptides can be harnessed to the specific recognition of non-biological entities and to the direction of metallic and semiconducting nano-particles synthesis (For a recent review see M. Sarikaya et al., 2003, Naturematerials 2: 577-585). Using combinatorial phage and cell-surface display libraries researchers were able to evolve peptides that bind selectively to different crystallographic planes of gold (Brown S., 2000, J. Mol. Biol. 299: 725-732), silver (Naik R.R. et al., 2002, Nature materials 1: 169-172), silica (Naik R.R. et al, 2002, J. Nanosci. Nanotechnol. 2: 1-6), zeolites (Scembri, M.A., et al., 1999, FEMS Microbiol. Lett. 170: 363-371), metal oxides (S. Brown, 1992, Proc. Natl. Acad. Sci. USA 89: 8651-8655; K. Kjacrgaard et al., 2000, Appl. Env. Microbiol. 66: 10-14), minerals (Gaskin, D.J.H., et al., 2000, Biotech. Lett. 22, 1211-1216), C60 (Braden, B. C. et al., 2000, PNAS 97 12193), carbon nanotubes (Wang, S., et al., 2003, Nat. Mat. 2: 196), and various semiconductors (Whaley, S. R. et al., 2000, Nature 405, 665 (2000); Lee, S-W., et al., 2002, Science 296: 892). The selected peptides displayed good discrimination between different crystal types and planes. Moreover some of them were found to catalyze the growth of specific crystal structures. The inspiration for these studies stems from protein guided mineralization in organisms and is hence denoted biomimetics. It is anticipated that the evolved peptides will facilitate defect free, low temperature synthesis and assembly of inorganic materials for nanotechnological applications. Indeed, very recently such peptides, displayed on a virus, were successfully utilized to direct synthesis of semiconducting and magnetic nano-wires (Mao C., et al., 2004, Science 303: 213-217).

It is noted that peptide selectivity to crystal structure and orientation implies that even atomically flat surfaces provide enough details for selectivity. It is important to appreciate that the distance between the binding sites on the peptide are longer than the crystal lattice constant. Several groups have demonstrated in recent years electrical control over the electrostatic interaction between surfaces. Using AFM to measure forces they could control the repulsion between a charged colloid and a surface, and even turn the repulsion into attraction. More recently, two groups' demonstrated electrical control over surface hydrophobicity and its turning into mild hydrophilicity. Electrical control over the electrostatic interaction between a charged colloid and a semiconducting surface is now available, tuning it continuously between repulsion and attraction.

Modeling of e.g. gold binding peptides teaches that the separation between binding sites typically measures 1-2 nanometers. Present fabrication technologies, most notably the Focused Ion Beam, are at the verge of this scale and may eventually provide such features. The more stringent constraint might potentially be a requirement for atomically accurate distance between any two binding sites.

However, selective binding of biomolecules to artificial substrates has been demonstrated only for peptides and in all of these reports the use of artificial surfaces has never been suggested for gaining electrical control over biological processes.

While reducing the present invention to practice, the present inventors have uncovered that individually contacted nano-scale electrodes, each biased to an individual set potential, can be used as artificial receptors capable of binding biological moieties and that such receptors can be used in any application which is based on molecular recognition involving electrostatic interactions such as to type ligands, identify binding domains of proteins and target delivery of drugs.

Thus, according to one aspect of the present invention there is provided an artificial receptor. The artificial receptor comprising a non-biological surface having an extent, the surface having unique surface electrical properties that vary over the extent, the electrical properties being such as to configure an electrical field about the surface to provide specific binding for a target biological moiety.

The phrase "artificial receptor" which is interchangeably used herein also as "an electrode" refers to a specific device made of a material (e.g., crystal) such as a semiconducting or a conducting material, which is configured to exhibit a unique surface electrical properties to provide specific binding as described hereinbelow for a target biological moiety.

The embodiments use electric fields which are configured to specifically bind biological moieties. A first embodiment has a permanent electric field pattern preset for a specific molecule. A second and a third embodiment are programmable to bind and release specific molecules at pre-determined times.

Referring now to the drawings, Figure 1 illustrates a first preferred embodiment of the present invention in which a device 10 is set up a surface 12 and an electric field about that surface. An isoelectric contour 14 illustrates a possible shape for the field.

Surface 12 of device 10 preferably includes a plurality of regions 16-26, each having a predetermined electrostatic field strength. Regions 16 - 26 within device 10 are constructed with built-in static electric fields that between them give the overall contour 14. The unique contour 14 is capable of binding a specific biological moiety as described hereinbelow (e.g., a protein). More particularly, the field binds a specific sub-region of the protein just as an antibody binds an antigen (e.g., epitope) or an enzyme's active site binds a corresponding site on a substrate.

It will be appreciated that since the specific interaction between biological moieties such as proteins often involves a unique three-dimensional binding site having a size of a few nanometers up to a few Angstroms, the regions defining the surface of the artificial receptor of the present invention are preferably in the Angstrom order of magnitude, more preferably, in the nanometer order of magnitude, so as to enable specific binding of biological moieties thereto.

Surface 12 of device 10 can be made of various materials having selected electrostatic properties. Non-limiting examples of such materials include ceramics and semiconductors (e.g., crystals or polycrystals such as PZT, GaAs and silicon). It will be appreciated that when a specific region is made of a crystal, the size of the region is preferably in the magnitude of five to ten lattice constants.

For example, device 10, is preferably constructed from ceramic bearing ferroelectric particles, allowing the ceramic to be pre- electrified with the desired field strength.

The result is an artificial receptor whose surface has defined and unique electrical properties that vary over its extent, the properties giving rise to an electrical field over the surface which provides specific binding for the target biological moiety.

It should be appreciated that the electrodes need not be planar. They may comprise, for instance, carbon nanotubes sticking out of the plane. The same is true for the gaps between electrodes.

Binding of the molecule to the surface is of a proximity and orientation which mimics the equivalent biological binding pair and the resultant affinity is of a K_{D} range of preferably 10⁻⁵-10⁻¹⁵ M, preferably at least 10⁻⁶ M, preferably at least 10⁻⁷ M, preferably at least 10⁻⁸ M, preferably at least 10⁻⁹ M, preferably at least 10⁻¹⁰ M preferably at least 10⁻¹¹ M, preferably at least 10⁻¹² M, preferably at least 10⁻¹³ M, preferably at least 10⁻¹⁴ M, preferably at least 10⁻¹⁵ M.

It will be appreciated that determination of such affinity can be performed using methods known in the art, such as, by scatchard analysis.

It will be appreciated that to enable the binding of a wide selection of molecules to the artificial receptor of the present invention and to control the binding of such molecules, the artificial receptor of the present invention is preferably programmable to provide different electrical fields as desired.

Such programming can be achieved by configuring at least one electrode capable of being biased to a unique electrical property.

An example for such configuration is presented in Figures 2a-d. A semiconductor wafer laminate 30 is constructed of layers of semiconductor. The layers are alternate conductors A and insulators B (e.g., GaAs/AlGaAs or metal/metal oxide; Figure 2a). The wafer may then be sliced along a transverse cross section to provide a surface in which the alternating conducting and insulating layers cross the width of the surface (Figure 2b). Figure 2c shows a magnified view of part of the length of the surface showing the alternating conducting layers A and insulating layers B. The cleaved surface comprises alternating strips with atomically sharp interfaces. To gain electrical control over the binding capacity of the artificial receptor of the present invention, the conducting layers are independently electrifiable layers. Thus, the user can set up any desired electrical field over the surface by controlling the electricity passed through each electrifiable layer.

Figure 2d illustrates an insulating coating layer 32, which may be applied on the surface of the artificial receptor. Cavitations 34, which are holes in the insulating coating allow the binding of the molecules to the surface. An example of coating layer 32 is a glass. Preferably cavitations 34 are in the Angstrom or nanometer scale.

The glass is preferably passivated against protein binding. Using conventional methods in microelectronics the A layers are all contacted electrically away from the cleaved edge. The result is a large array of cavitations in the coating layer, each over an A/B interface. The conducting, A side of all spots can then be biased relative to the solution. The exposed A/B interface is the target for the antibodies. Since the peptide binding sites are typically either charged or polarized, the local electrostatic potential created by a different biasing of the A electrode should affect differently various antibody molecules.

The surface of the artificial receptor may comprise zero dimension, one dimension, two dimensions and three dimensions.

The surface of the artificial receptor may comprise electronically controllable hydrophobic coating to allow controllable hydrophobic properties for each independently electrifiable layer.

According to preferred embodiments of the present invention, variable electrical fields are applied to the regions. Such electrical fields can be controlled by adjusting the voltage and/or current supplied to the electrode or the layer as described hereinabove and those of skills in the art are capable of controlling the electrical fields.

Thus, the electrically biased artificial receptor is capable of binding various targets depending on the resultant electrical field provided at each time.

According to preferred embodiments of the present invention, the surface comprises switchable wiring, such wiring being switchable to provide the unique electrical properties. Preferably, such wiring is variably switchable, thereby providing specific binding to different target biological moieties as desired.

Reference is now made to Figure 3, which is a simplified diagram showing a preferred switching device for switching the different layers as desired.

Preferably, the wafer layers are connected to the switching device or selector 38, which allows voltage levels to be electronically selected for each of the independently electrifiable layers. The electrical field may thus be varied to provide specific binding to different targeted biological moieties. Typically an overall power source 40, which may be a battery or a main fed power supply, supplies a series of voltage regulated power sources 42.1...42.n, each set at different voltage levels. A switching matrix 44 then connects any one of the layers 46.1...46.n to any one of the regulated power sources. Preferably the switching matrix is controlled by software. It will be appreciated that the switching device described with respect to Figure 3 is merely an example and other alternatives will occur to the skilled person.

An alternative approach to control the microscopic electric field landscape of the artificial receptor of the present invention relies on the use of materials which upon application of an electric field, their unit cell or molecular structure is changed, resulting in a change in the electric field produced thereon. Such materials are for example, ferroelectric substances (e.g., PLZT) which exhibit high dielectric constants and are therefore changeable following application of changes in their electric field,

One example for a ferroelectric material is a Perovskite-like crystal, in which a high valence cation is encapsulated in an oxygen octahedron. The oxygen together with the A atoms form an face center cubic (fcc) crystal with the latter atoms at the corners. The high temperature phase is cubic and, hence, lacks electric moments. As the temperature is reduced the material may undergo a series of structural phase transitions to lower symmetry structures accompanied by large local electric moments. Since the central cation has a large charge and relatively broad energy minima, the electrical susceptibility is very large and the dielectric constant can approach values as high as 1000-5000. The corresponding polarization fields are enormous. One such crystal, PLZT, is particularly attractive for the scope of the present invention. At a ratio of 9/65/35 the virgin ceramics maintains an isotropic cubic phase. An application of a moderate electric field shifts the crystal to the rhombohedral or tetragonal phases characterized by enormous local electric dipoles. When the external field is removed the polar phase relaxes instantaneously back to its unpolarized cubic phase. The magnitude of the generated dipoles depends on the applied field. The large dielectric constant guarantees extremely large local electric moments.

For example, as described in Figure 5, PLZT crystal 64 can be placed between a metal cathode 60 and a metal anode 62. Antibodies which selectively bind to PLZT subjected to a certain field can be easily identified.

In the preferred embodiment, different target molecules (e.g., antibodies) bind selectively to the same crystal under different applied fields and released under other fields.

The physics of PLZT is fully understood and the phase diagram as a function of composition, temperature, and applied field is tabulated.

It will be appreciated that the surface of the artificial receptor of the present invention can be further modified by attaching materials or molecules capable of modifying the electrical property of the surface, as well as the hydrophilic or hydrophobic properties of the surface which may affect the capacity of the surface to form hydrophobic interactions, hydrogen bonding and van der Waals interactions with biological moieties.

Several types of substances and molecules can be used to change the electric filed of the surface according to this aspect of the present invention. These include molecules and materials which following the application of an electric field, mechanical stress and/or change in a temperature are capable of modifying the electric field generated thereupon. Substances which may affect the hydrophobic or hydrophilic properties of the surface may be, for example, charged peptides, phospholipids and the like, which following the application of an electric field can fold or change their relative orientation with respect to the surface.

For example, organic molecules such as hydroquinone, Rotaxane and charged organic or biological polymers, undergo atomic and/or molecular changes following the application of an electric field. Figures 6 and 7 illustrate the molecular and structural changes occurring following the application of an electric field on hydroquinone and Rotaxane, respectively.

Hydroquinone looses two hydrogen atoms following the application of an electric filed. The hydroquinone molecule may be attached to a substrate via an alkane tail. In either case the molecule may be switched between two stable states by electro-protonation. An antibody molecule selective to one of the configurations is attracted or released from the Hydroquinone depending on its state. The Hydroquinone transduces in this case the electronic signal to a change that is readily recognizable by antibodies.

Rotaxane is a linear dumbbell shaped molecule inserted into a mobile molecular ring having two redox states. The ring may rest in one of two positions along the molecule depending on the oxidation state. The latter is controlled by application of a bias between the substrate to which the dumbbell molecule is bound and the solution. It is very likely that antibodies can be selected to the two different configurations of the molecule, namely for the two positions of the ring along the molecule, hence providing an electrical control over which antibody binds the surface.

The ferroelectric materials (e.g., PLZT) which are described hereinabove represent an example of non-organic materials which upon the application of electric field exhibit structural changes that affect the electric field and, hence, the target for antibodies and other bio-molecules.

Pyroelectric materials can also be used to modify the electric field of the surface of the artificial receptor of the present invention. Following the application of a temperature change, the material undergoes structural changes of the unit cell or a molecular change which result in a modified electric field. Non-limiting examples for such materials which can be used along with the present invention include, Li-Ta-O₃ and triglycine sulfate (TGS).

Piezoelectric materials can also be used to modify the electric field of the surface of the artificial receptor of the present invention. Following the application of electric voltage, the material undergoes conformational changes which result in a change in the unit cell. Reversal to the original conformation usually requires the application of an opposite voltage since the material is somewhat hysteretic. Non-limiting examples for such materials which can be used along with the present invention include, PZT and polyvinylidene fluoride (PVDF).

Ferroelastic materials can be also used to modify the electric field landscape of the surface of the artificial receptor of the present invention. Following the application of mechanical stress, the material undergoes conformational changes which result in a change in the electric field. A non-limiting example for such materials which can be used along with the present invention is LaAlO₃.

It will be appreciated that several biological moieties can be also attached to the surface of the artificial receptor of the present invention in order to modify its electric field. For example, peptides can be attached to the surface and following the application of an electric field the peptide can change its configuration, e.g., can form a cyclic molecule or can attach along the surface. Such a change in configuration results in a change of the electrical properties of the surface or hydrophobic nature, or display of certain groups which can be used to selectively bind biological moieties.

It will be appreciated that such materials and substances which following structural and molecular changes (as a result of mechanical stress, temperature change and/or electric field change) are capable of modifying their electric field can be used to form the regions of the artificial receptor instead of the crystals or semiconductors.

While further reducing the present invention to practice, the present inventors have devised an artificial receptor having a surface with switchable electrical conductive tracks, the conductive tracks being switchable to configure an electrical field about the surface to provide specific binding for a target biological moiety.

According to preferred embodiments of the present invention, the artificial receptor further comprises a switching control for switching the conductive tracks.

Reference is now made to Figure 4, which is a simplified diagram illustrating a further preferred embodiment of the present invention. The device of Figure 4, like the device of Figures 2a-d is a programmable device so that the electrical fields produced can be changed during use. Device 50 comprises a conventional semiconductor wafer surface 52 on which are patterned conductive tracks 54 using conventional semiconductor manufacturing techniques. The conductive tracks are switched using transistors in the conventional manner. It is stressed that in a standard semiconductor integrated circuit, electrical fields are produced and are generally a nuisance, giving rise to various unwanted phenomena as stray or parasitic capacitance, which slow down the propagation rates of the leads, and introduce noise and interference between the components. The present embodiments however make use of the field to target the desired molecules.

Device 50 may include additional elements such as a covering layer over the semiconductor surface, as described above.

### Elaborate artificial receptor (electrode) set

It will be appreciated that for activation or suppression of a single bio-process or even for the selection between two bio-processes a single electrode with two states is enough. For selection between multiple pathways or activation/suppression of multiple processes an elaborate electrode set with numerous biasing configurations may be required. The electrode size and separation may vary according to the application. Electrodes larger than ∼ 10 nanometer may be fabricated by conventional methods of micro and nanoelectronics such as electron beam lithography and focused ion beam. Smaller electrodes and/or smaller spacing between electrodes, still with an individual electrical contact to each of the electrodes can be realized by the utilization of metal/metal oxide alternating layers grown by molecular beam epitaxy. Separate contacts to the individual layers can be achieved by using a moving shutter during growth and later use of photolithography to contact the exposed metallic layers, this being illustrated in Figures 8a-d. Figures 8a-d are successive stages in the manufacture of the electrode layers, showing how the individual conducting layers can each have independent electrical connections. Fig 8a shows an initial stage in which shutter 70 is located at the far left side of wafer 72, allowing deposition of a pair 74 of successive conducting and insulating layers. Moving to Fig. 8b and the shutter 70 is moved, say by 100 micrometers to the right and a further pair 76 of conducting and insulating layers is deposited. The shutter 70 is then moved further to the right in Fig. 8c and two more layers 78 are deposited. In Fig. 8d, a further pair 80 of layers is deposited with the shutter 70 moved even further to the right. The resulting structure has terraces spaced by say 100 micrometer, each exposing a conducting layer. Using conventional photolithography, each such layer can be electrically contacted independently. Upon cleavage of the substrate perpendicular to the deposition direction, the layers are exposed in the form of thin lines, each contacted separately. Modem Molecular Beam Epitaxy technology facilitates fabrication of layers as thin as two monolayers spaced by an insulating layer of a comparable thickness. After cleavage these dimensions translate to conducting or electrode layers which are two monolayers thick, separated by similar insulating layers.

An alternative approach relies on extension of the distance between the biological binding sites. The simplest construction would be an IgG antibody composed of two halves, each selective to a different bias. Such a construction should be selective to two electrodes spaced by a few nanometers. If two antibodies are fused at their tails (as occurs naturally with IgA) that distance can increase to 10-20 nm. The latter distance is easily accessible by present nanotechnology and it should be possible to contact independently two interfaces spaced by such a distance. One may obviously utilize other artificial or natural constructs like IgM pentamers.

### Floating electrodes

The electrodes as described above need not necessarily be fabricated on a supporting substrate nor need they be exclusively biased by an external power supply, although in several preferred embodiments they are so biased. In one embodiment the electrodes may comprise a microscopic p-n junction realized in a semiconducting nanocrystal. Due to the rectifying nature of such a junction, application of radiation to the nanocrystal results in generation of bias between the two poles of the p-n junction. This bias is equivalent to an external bias applied to electrodes by connecting them to an external source. The possibility to apply bias from a distance by radiation at a desired timing facilitates, for instance, drug release by external radiation. For that the drug is fused to the antibody and the latter is released by the induced voltage as described above.

Other bias sources include, e.g. a microscopic battery or even a chemical cell whose output depends on certain biochemical parameters, such as pH. A source like that may trigger antibody binding or release from the electrodes based on the presence of certain chemicals. Such an arrangement facilitates e.g. localized drug release depending on certain biochemical conditions.

The devices (or the electrodes) described hereinabove can be connected together in any desired way in order to provide three-dimensional fields. Furthermore, numerous devices (or electrodes) may be connected together to form arrays. Thus, the electrodes in the array may be identical, designed to fish out the maximum number of a target molecule, or the arrays may comprise electrodes with different fields or electrodes programmed differently, so that a range of target molecules can be searched for.

The following section describes possible embodiments of the electrodes set. The processes mentioned in the text are known to practitioners in the fields of micro and nanoelectronics, crystal growth, surface chemistry, etc.

***Electrodes material -*** The electrode material used by the method of the present invention can be any type of material or materials combination. Following is a list of preferred materials:
***(a) Metal electrodes -*** deposited on an insulating substrate (planar geometry) on a substrate such as glass, alumina, sapphire, etc. Examples for metals include gold, platinum, silver, aluminum, etc. The electrodes may be defined either by first depositing or epitaxially growing a metal layer (e.g. by molecular beam epitaxy, chemical vapor deposition, atomic layer deposition, electrochemistry) and then patterning it by e.g. electron beam lithography or focused ion beam (FIB). Alternatively, the electrodes may be deposited or grown on the substrate already in their patterned form, for instance by patterned epitaxial growth or by FIB deposition.
***(b) Semiconductor electrodes -*** deposited on an insulating substrate (planar geometry) on a substrate such as glass, alumina, sapphire, etc. Examples for semiconductors include silicon, GaAs, InAs, CuO, etc. The electrodes may be defined either by first depositing or epitaxially growing a semiconductor layer (e.g. by molecular beam epitaxy, chemical vapor deposition, atomic layer deposition, electrochemistry) and then patterning it by e.g. electron beam lithography or focused ion beam (FIB). Alternatively, the electrodes may be deposited or grown on the substrate already in their patterned form, for instance by patterned epitaxial growth or by FIB deposition.
***(c)*** ***Conducting polymers electrodes** -* deposited on an insulating substrate (planar geometry) on a substrate such as glass, alumina, sapphire, etc. Examples for conducting polymers include PPV, polyanilin, etc. The electrodes may be defined either by first depositing or epitaxially growing a polymer layer (e.g. by molecular beam epitaxy, chemical vapor deposition, atomic layer deposition, electrochemistry) and then patterning it by e.g. electron beam lithography or focused ion beam (FIB). Alternatively, the electrodes may be deposited or grown on the substrate already in their patterned form, for instance by patterned epitaxial growth or by FIB deposition. The conducting polymers may be deposited or grown either parallel to the surface or angled to it.
***(d) Semiconductor superlattice (vertical geometry) -*** Alternating layers of various semiconductor materials are grown on a substrate. In the simplest embodiment depicted in Figures 2a-d the superlattice comprises two alternating layers of conducting and insulating semiconductors. In another realization the structure may comprise an elaborate sandwich of different materials. The structure may be grown by molecular beam epitaxy (MBE), chemical vapor deposition (CVD), metalo-organic molecular beam epitaxy (MOMBE), liquid phase epitaxy (LPE), chemical deposition, electrochemistry, atomic layer deposition, etc. The wafer is then cleaved as described in Figure 1b and the layers exposed by the cleavage serve as electrodes. The layers may be crystalline, amorphous, polycrystalline, or combinations of the above.
***(e) Metal*/*insulator superlattice (vertical geometry) -*** Alternating layers of various metals and insulating layers, e.g. metal oxides or ceramics, are grown on a substrate. In the simplest embodiment depicted in Figure 5 the superlattice comprises two alternating layers of metal and insulating metal oxide. In another realization the structure may comprise an elaborate sandwich of different materials. The structure may be grown by molecular beam epitaxy (MBE), chemical vapor deposition (CVD), metalo-organic molecular beam epitaxy (MOMBE), liquid phase epitaxy (LPE), chemical deposition, electrochemistry, atomic layer deposition, etc. The wafer is then cleaved as described in Figures 2a-d and the layers exposed by the cleavage serve as electrodes. The layers may be crystalline, amorphous, polycrystalline, or combinations of the above.
***(f) Conducting polymers (vertical geometry) -*** Alternating layers of various conducting polymers and insulating layers, e.g. metal oxides, ceramics, or insulating molecules and polymers are grown on a substrate. The structure may be grown by molecular beam epitaxy (MBE), chemical vapor deposition (CVD), metalo-organic molecular beam epitaxy (MOMBE), liquid phase epitaxy (LPE), chemical deposition, electrochemistry, atomic layer deposition, etc. The wafer is then cleaved as described in Figures 2a-d and the layers exposed by the cleavage serve as electrodes. The layers may be crystalline, amorphous, polycrystalline, or combinations of the above.
***(g) Molecular conductors and semiconductors -*** like carbon and other nanotubes provide in a natural way nanometer scale electrodes. Carbon nanotubes can be spin coated on an insulating layer or grown from metal catalysts. A particular attractive approach in the growth of multiple tubes from patterned catalysts islands. Other molecular conductors like semiconductor nanowires, nanorods, and dots can be used. The latter include also self assembled semiconductor dots defined by strain on a semiconductor substrate.
***(h) Functionalized or unfunctionalized biomolecules -*** see E. Braun, Y. Eichen, U. Sivan and G. Ben Yoseph, DNA templated assembly and electrode attachment of conducting silver wire, Nature, 391, 775 (1998); K. Keren, M. Krueger, R. Gilad, G. Ben-Yoseph, U. Sivan and E. Braun, Sequence-Specific Molecular Lithography on Single DNA Molecules, Science, 297, 72 (2002); and K. Keren, R. S. Berman, E. Buchstab, U. Sivan, and E. Braun, DNA-Templated Carbon-Nanotube Field Effect Transistor, Science 302, 1382 (2003), which are fully incorporated herein by reference.

***Ferroelectric and high dielectric constant electrodes -*** Ferroelectric and high dielectric constant ceramics and organics provide an efficient way for the creation of large local electric moments. The principle is presented hereinabove and in Figure 5. Multiple electrode sets can be fabricated either in a planar geometry or a vertical geometry as described hereinabove.

***Electrode functionalization and coating -*** The electrodes can be functionalized by biological, non-biological, or organic molecules. The latter may serve to modify the surface properties such as hydrophobicity/hydrophilicity, charge, stability, roughness, compatibility with the solutions and the molecules in solution, non-specific binding, etc. Functionalization may also provide an electronic control over surface properties as detailed e.g. in Frechette and Vanderlick, Langmuir 17, 7620 (2001); Barten et al. Langmuir 19, 1133 (2003). The electrodes can also be coated with polymers, gels, etc. for protecting them or the antibodies against chemical processes such as oxidation or reaction and for increasing the effective electrode area. Certain substances such as agarose provide a convenient environment to the biomolecules. The electrodes can also be modified with thin insulating layers such as silica and alumina. They can also be covered with colloids and beads.

***Contacting individual electrodes -*** Individual electrodes are contacted by conventional microelectronics techniques. In the case of semiconductor superlattice access to the individual conducting layers may be provided either by post-growth selective etching or by masking parts of the layers during the crystal growth as depicted in Figure 8. The exposed conducting layers are contacted by well-established methods in microelectronics.

It will be appreciated that the artificial receptor surfaces of the present invention can interact with the binding molecules. Such interaction can be monitored by an atomic force microscope (AFM) adapted for force measurements. The antibody or peptide are attached to the AFM tip and substrate, respectively, and the tip deflection is monitored as a function of the separation between the tip and the artificial receptor for different fields applied between the substrate and/or tip and the solution. Since the tip spring constant is measured independently, the deflection can be translated directly to force.

Thus, as described hereinabove, the artificial receptor of the present invention can be configured using any material/substance and method known in the art.

For example, as is shown in Table 2 and is described in Example 1 of the Examples section which follows, while the GaAs (100) surface specifically bound clone No. F10, the GaAs (111A) surface specifically bound clones El, F1 and C7. Thus, the predetermined electrostatic field of the GaAs crystal when cut at the 100 plane is different than that formed on the 111A plane and thus, various biological moieties specifically bind to each predetermined electrostatic field.

It will be appreciated that variation of the electrical field as described hereinabove is likely to alter the binding capacity of the surface, thus resulting in different biological moieties attaching thereto.

Thus, the teachings of the present invention can be used to gain electrical control over biological processes, namely, to trigger or suppress a selected biological pathway by an electronic signal presented to the system. A given antibody can bind a given set of electrodes biased to a certain voltage pattern and avoid binding to the same electrode set when biased in a different pattern. The latter pattern may, in turn, attract a different antibody. The same set of electrodes biased in different ways thus specifically bind different target molecules from the solution and, hence, act as a programmable artificial receptor.

Thus, the artificial receptor of the present invention can be used to provide specific binding for a target biological moiety.

The phrase "specific binding" as used herein, refers to binding of a biological moiety via electrostatic, hydrophobic, hydrogen bonds and van der Waals interactions to an artificial receptor having a surface with unique electrical properties.

As used herein, the phrase "biological moiety" refers to any naturally occurring or synthetic macromolecule having a biological function. Examples include, but are not limited to DNA, RNA, protein, peptide (e.g., antigen, epitope), carbohydrate, antibodies and fragments thereof. It will be appreciated that the biological moiety used by the present invention can be isolated or included in a prokaryotic (e.g., bacteria, viruses) or eukaryotic organism (e.g., mammals). Nonlimiting examples of biological moieties which can be used along with the present invention, include, growth factors, cytokines, transcription repressors, transcriptions enhancers, promoters (e.g., DNA, RNA and/or proteins), and any other molecule which can trigger, suppress, control or regulate any biological process.

The term "antibody" as used in this invention includes intact molecules as well as functional fragments thereof, such as Fab, F(ab')2, Fv or single domain molecules such as VH and VL to an epitope of an antigen. These functional antibody fragments are defined as follows: (1) Fab, the fragment which contains a monovalent antigen-binding fragment of an antibody molecule, can be produced by digestion of whole antibody with the enzyme papain to yield an intact light chain and a portion of one heavy chain; (2) Fab', the fragment of an antibody molecule that can be obtained by treating whole antibody with pepsin, followed by reduction, to yield an intact light chain and a portion of the heavy chain; two Fab' fragments are obtained per antibody molecule; (3) (Fab')2, the fragment of the antibody that can be obtained by treating whole antibody with the enzyme pepsin without subsequent reduction; F(ab')2 is a dimer of two Fab' fragments held together by two disulfide bonds; (4) Fv, defined as a genetically engineered fragment containing the variable region of the light chain and the variable region of the heavy chain expressed as two chains; (5) Single chain antibody ("SCA"), a genetically engineered molecule containing the variable region of the light chain and the variable region of the heavy chain, linked by a suitable polypeptide linker as a genetically fused single chain molecule; and (6) Single domain antibodies are composed of a single VH or VL domains which exhibit sufficient affinity to the antigen.

Methods of producing polyclonal and monoclonal antibodies as well as fragments thereof are well known in the art (See for example, Harlow and Lane, Antibodies: A Laboratory Manual, Cold Spring Harbor Laboratory, New York, 1988, incorporated herein by reference).

Antibody fragments according to the present invention can be prepared by proteolytic hydrolysis of the antibody or by expression in E. coli or mammalian cells (e.g. Chinese hamster ovary cell culture or other protein expression systems) of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')2. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly. These methods are described, for example, by Goldenberg, U.S. Pat. Nos. 4,036,945 and 4,331,647, and references contained therein.
See also Porter, R. R. [Biochem. J. 73: 119-126 (1959)]. Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introduction of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10,: 779-783 (1992); Lonberg et al., Nature 368: 856-859 (1994); Morrison, Nature 368 812-13 (1994); Fishwild et al., Nature Biotechnology 14, 845-51 (1996); Neuberger, Nature Biotechnology 14: 826 (1996); and Lonberg and Huszar, Intern. Rev. Immunol. 13, 65-93 (1995).

The term "peptide" as used herein encompasses native peptides (either degradation products, synthetically synthesized peptides, or recombinant peptides), peptidomimetics (typically, synthetically synthesized peptides), and the peptide analogues peptoids and semipeptoids, and may have, for example, modifications rendering the peptides more stable while in a body or more capable of penetrating into cells. Such modifications include, but are not limited to: N-terminus modifications; C-terminus modifications; peptide bond modifications, including but not limited to CH₂-NH, CH₂-S, CH₂-S=O, O=C-NH, CH₂-O, CH₂-CH₂, S=C-NH, CH=CH, and CF=CH; backbone modifications; and residue modifications. Methods for preparing peptidomimetic compounds are well known in the art and are specified, for example, in Ramsden, C. A., ed. (1992), Quantitative Drug Design, Chapter 17.2, F. Choplin Pergamon Press, which is incorporated by reference as if fully set forth herein. Further details in this respect are provided hereinbelow.

The peptides of the present invention may be synthesized by any techniques that are known to those skilled in the art of peptide synthesis. For solid phase peptide synthesis, a summary of the many techniques may be found in: Stewart, J. M. and Young, J. D. (1963), "Solid Phase Peptide Synthesis," W. H. Freeman Co. (San Francisco); and Meienhofer, J (1973). "Hormonal Proteins and Peptides," vol. 2, p. 46, Academic Press (New York). For a review of classical solution synthesis, see Schroder, G. and Lupke, K. (1965). The Peptides, vol. 1, Academic Press (New York).

Further description of peptide synthesis is disclosed in U.S. Pat. No. 6,472,505. A preferred method of preparing the peptide compounds of the present invention involves solid-phase peptide synthesis, utilizing a solid support. Large-scale peptide synthesis is described by Andersson Biopolymers 2000, 55(3), 227-50.

The DNA or RNA molecules of the present invention can be used in the form of oligonucleotide or polynucleotide molecules.

The term "oligonucleotide" refers to a single-stranded or double-stranded oligomer or polymer of ribonucleic acid (RNA) or deoxyribonucleic acid (DNA) or mimetics thereof. This term includes oligonucleotides composed of naturally occurring bases, sugars, and covalent internucleoside linkages (e.g., backbone), as well as oligonucleotides having non-naturally occurring portions, which function similarly to respective naturally occurring portions.

Oligonucleotides designed according to the teachings of the present invention can be generated according to any oligonucleotide synthesis method known in the art, such as enzymatic synthesis or solid-phase synthesis. Equipment and reagents for executing solid-phase synthesis are commercially available from, for example, Applied Biosystems. Any other means for such synthesis may also be employed; the actual synthesis of the oligonucleotides is well within the capabilities of one skilled in the art and can be accomplished via established methodologies as detailed in, for example: Sambrook, J. and Russell, D. W. (2001), "Molecular Cloning: A Laboratory Manual"; Ausubel, R. M. et al., eds. (1994, 1989), "Current Protocols in Molecular Biology," Volumes I-III, John Wiley & Sons, Baltimore, Maryland; Perbal, B. (1988), "A Practical Guide to Molecular Cloning," John Wiley & Sons, New York; and Gait, M. J., ed. (1984), "Oligonucleotide Synthesis"; utilizing solid-phase chemistry, e.g. cyanoethyl phosphoramidite followed by deprotection, desalting, and purification by, for example, an automated trityl-on method or HPLC.

The oligonucleotide of the present invention is of at least 5, at least 6, at least 7, at least 8, at least 9, at least 10, at least 11, at least 12, at least 13, at least 14, at least 15, at least 16, at least 17, at least 18, at least 19, at least 20, at least 22, at least 25, at least 30 or at least 40, bases specifically hybridizable with sequence alterations known in the art.

The oligonucleotides of the present invention may comprise heterocylic nucleosides consisting of purines and the pyrimidines bases, bonded in a 3'-to-5' phosphodiester linkage.

Oligonucleotides are may be modified either in backbone, internucleoside linkages, or bases, using methods known in the art.

For example, the teachings of the present invention can be used to identify ligands of specific artificial receptors.

Thus, according to yet another aspect of the present invention there is provided a method and a kit for typing ligands.

As used herein, the phrase "typing ligands" refers to identifying ligands, *i.e*., biological moieties as described hereinabove which are capable of binding a specific surface or region of the artificial receptor of the present invention.

"Identifying" according to this aspect of the present invention refers to determining the amino acid sequence, nucleic acid sequence, and/or carbohydrate structure of the biological moiety which binds to the artificial receptor of the present invention. It will be appreciated when phage display libraries are utilized, determination of the DNA sequence of the clones generating the displayed peptide or protein molecules is preferably effected.

The method is effected by subjecting the ligands to a plurality of the artificial receptor of the present invention using conventional screening or panning methods which are optimized to fit the artificial receptor of the present invention. Thus, the kit for typing ligands further includes reagents (as described hereinbelow) for qualifying binding of the ligands to the plurality of artificial receptors.

Phage display is a powerful technology designed to evolve, from an initial library, peptides and antibody fragments having high affinity to a certain antigen. The most widely used library methodology is based on the filamentous phage, a bacteriophage that infects male Escherichia coli. Filamentous phage display is based on cloning DNA fragments encoding millions of variants of certain ligands (e.g. peptides, proteins or fragments thereof) into the phage genome, fused to the gene encoding one of the phage coat proteins (usually pIII, but also pIV, pVI or pVIII). Upon expression, the coat protein fusion is incorporated into new phage particles that are assembled in the periplasmic space of the bacterium. Expression of the gene fusion product and its subsequent incorporation into the mature phage coat results in the ligand being presented on the phage surface, while its genetic material resides within the phage particle. This connection between genotype and phenotype allows the enrichment of specific phage by selection on an immobilized affinity target. The phages are caused to interact with the target antigen while the latter is immobilized. Phages that display a relevant ligand are retained by virtue of their binding to the target, while non-adherent phages are washed away. Bound phages are recovered from the surface, used to re-infect bacteria and reproduced for further enrichment followed by another affinity assay. With an appropriate starting library, several such cycles usually lead to satisfactory selectivity and binding affinity.

Antibodies selected from phage libraries may not be optimal for direct application. In many cases, manipulation of the antibody affinity, valency, specificity, or stability is required. In this case, phage display technology is applied in a manner similar to the production of synthetic libraries and selection of the best binders from them. Such secondary libraries contain variants of the antibodies isolated initially with mutations introduced either randomly or following a rational design. Mutations are introduced into the antibody genes using one of several methods: site-directed mutagenesis, error-prone PCR, chain shuffling, DNA shuffling, or mutator E. coli strains. Using one of these approaches it is possible to obtain antibodies having high affinities, (for biological antigens these methods yield affinities well below 100 pM) and good selectivity.

Any method that separates phages that bind from those that do not, can be used for phage selection, and indeed, many different selection methods can be used. The popular selection methods include affinity selection (also called biopanning) on immobilized antigen coated onto solid supports, columns or BIAcore sensor chips.

Antibodies in the form of recombinant antibody fragments were the first proteins to be successfully displayed on the surface of a phage. This was achieved by fusing the coding sequence of the antibody variable (V) regions encoding for a single-chain Fv (scFv) fragment to the amino terminus of the phage gene III, coding for the phage minor coat protein pIII. Initial attempts to display Fab' fragments fused to pVIII, the phage major coat protein, were also successful. However, the pVIII site, although very popular for peptide phage display, is not suitable for the efficient display of large polypeptides such as antibodies. For this reason, most antibody phage-display systems utilize the pIII site.

Antibodies were first displayed using a phage vector, based on the genome of fd-tet and its gene III as fusion partner. In this vector, the genes coding for antibody scFv fragments were cloned in-frame with gene III and downstream of the gene III signal sequence, which normally directs the export of the phage-coat protein to the periplasm. Here, the antibody VH and VL domains may fold correctly, both stabilized by an intramolecular disulfide bridge, and pair to form a functional scFv.

The success of ligand phage display hinges on the synthesis of a large combinatorial repertoire on the phage and efficient selection and enrichment strategies.

### Affinity selection of initial library (biopanning)

For affinity selection, the phage display antibody library is exposed to the artificial receptor or the array of electrodes described hereinabove. The following description refers to one embodiment of the present invention. In a first step, antibodies selective to the artificial receptor of the present invention such as the structure depicted in Figure 2d, under a given bias relative to the solution, are selected from the phage display library described hereinabove. For example, the selection preferably contains a collection of molecules each selective to either the A material, the B material, or the A/B interface, all under the given bias. Antibodies specific for either A or B are selected by interacting the same library with pure A or pure B crystals under the same biasing condition. Antibodies selective to the A/B interface are selected by reacting the antibodies selected on the structure depicted in Figure 2d with pure A and then pure B biased crystals. Specific binding to these crystals depletes the collection from A and B binders, leaving mostly A/B specific binders. More than one selection round may be needed.

It will be appreciated that blocking of non-specific binding to the artificial receptor of the present invention can be achieved by incubating the artificial receptor with conventional blocking reagents such as bovine serum albumin, goat serum albumin or milk (e.g., 1 %).

As described in Example 1 of the Examples section which follows, to increase specificity of binding to the artificial receptor of the present invention, depletion cycles are preferably effected prior to panning. For depletion, the electrodes of the artificial receptor are preferably switched or biased with a different voltage than provided for the panning. As for the semiconductor surfaces described in Example 1 of the Examples section which follows, no wash step is needed between the depletion step and the subsequent panning step.

Tuning washing times and stringency helps to determine the selection efficiency and to discriminate between phages with different affinities for the target. At times it pays to perform the initial rounds of selection under low stringency, so as not to lose rare binders, and to employ more stringent conditions in later rounds.

Elution of bound phages from the artificial surfaces of the present invention can be effected using basic (e.g., TEA at pH 12) or acidic (e.g., Glycine-HCl, at pH 2.2) conditions, depending on the surfaces used (for details see Example 1 of the Examples section which follows). Alternatively, elution of bound phages from the electrifiable electrodes or the biased artificial receptor of the present invention can be effected by simply switching the wiring or varying the voltage supplied to the electrodes. Thus, a specific biased surface which specifically binds a ligand can be biased to release such ligand into the solution to thereby elute the desired phage display antibody.

Binding of scFv-displaying phage in ELISA can be conventionally detected by primary rabbit anti-M13 antisera in combination with a horseradish peroxidase (HRP) conjugated anti-rabbit antibody. Alternatively, an HRP-anti-M13 conjugate may be used. As is shown in Examples 1 and 3 of the Examples section which follows, the same methods can be used on the artificial receptor surfaces of the present invention. Polyclonal phage ELISA on biased A, B, and A/B crystals can be performed to differentiate between specific and non-specific binders as well as for identification and quantification of the various selective binders.

### Monoclonal Phage ELISA

To identify monoclonal phage antibodies the phagemids need to be rescued individually. Growth of phagemid containing cells and helper phage rescue is carried out in sterile 96 well, flat bottomed tissue-culture plates, essentially as described Example 1 of the Examples section which follows. In principle, colonies picked from the last panning cycle are expected to yield the most positive binders. However, it will be appreciated that since in the late cycles the phage population becomes dominated by a few (or even one) binders, clones from the outputs of earliest panning cycles that test positive in the polyclonal phage ELISA are also preferably selected for further analysis.

### Production and ELISA analysis of soluble scFvs

This test should be carried out in parallel with phage ELISAs to analyze individual clones for antigen binding. The phages need to be infected into HB2151, that does not carry an amber suppressor tRNA and then induced to give soluble expression of antibody fragments for ELISA.

### Second generation libraries

Antibodies selected from phage libraries may not be optimal for direct application. In many cases, manipulation of the antibody affinity, valency, specificity, or stability is required. In this case, phage display technology is applied in a manner similar to the production of synthetic libraries and selection of the best binders from them. Such secondary libraries contain variants of the antibodies isolated initially with mutations introduced either randomly or following a rational design. Mutations can be introduced here into the antibody genes using one of several methods: site-directed mutagenesis, error-prone PCR, chain shuffling, DNA shuffling, or mutator E. coli strains. Using one of these approaches it should be possible to obtain antibodies having high affinities, (for biological antigens these methods yield affinities well below 100 pM) and good selectivity.

### Antibodies for different bias values

The same procedure is applied to the same multi-layer structure biased at different potentials relative to the solution. An optimal antibody for a certain bias might turn an excellent starting point for the generation of a phage display library to be screened by the same interface biased differently. At each bias, antibodies for the A and B crystals as well as for the A/B interface are evolved as described above. The binding regions are sequenced and compared. The sequences displayed by antibodies optimized for different bias values differ from each other. It is extremely interesting to test how large the bias difference should be in order to obtain two different binding regions for two bias values. Strong affinity to a certain bias value is just one aspect of the desired antibodies. The complimentary aspect is selectivity which can be improved by interacting good binders for a certain bias with the same structure biased to a different value. Antibodies that bind also to a different bias can be depleted this way from the library (for further description of depletion steps, see Example 1 of the Examples section which follows).

It will be appreciated that the end result of the screening described hereinabove is a collection of vials, each containing monoclonal antibodies with optimal selectivity to one of the biased surfaces of the artificial receptor of the present invention, e.g., the A, B, or A/B structures as described in Figure 2 or the PLZT crystal described in Figure 5 when subjected to a given electric field.

### Screening of carbohydrate ligands using the artificial receptors of the present invention

Due to their inherent bond type complexity, synthesizing complex carbohydrate combinatorial libraries necessitates a plurality of distinct synthesis reactions. Carbohydrate libraries can be synthesized employing the "one bead-one molecule" approach, in which the diversity is created by a split-and-pool synthesis or the dynamic combinatorial chemistry (DCC) approach (see for example Schullek JR, et al., 1997, Anal. Biochem, 246: 20-9; U.S. Pat. Appl. No. 20040146941 to Zhang Biliang et al; Ramstrom O, Lehn JM. Chembiochem. 2000 1: 41-8 ). Such libraries can be screened on the artificial surface of the present invention such that the carbohydrate structures interact with a specific surface are identified, isolated and analyzed for composition.

It will be appreciated that the ligands and corresponding artificial receptors can be used in various biological applications. For example, such a ligand and artificial receptor can be used for targeting delivery of a drug molecule.

Thus, according to an additional aspect of the present invention there is provided a method of controlling a delivery of a drug molecule to a tissue of a subject. The method is effected by (a) implanting a device body including at least one electrode configured capable of a unique surface electrical property enabling a specific binding of a ligand thereto, the ligand being attached to the drug; (b) modifying the unique surface electrical property to thereby-control a binding or a release of the ligand and thereby controllably deliver the drug molecule to tho tissue.

As used herein the term "implanting" refers to administering the artificial receptor of the present invention to a subject in need thereof, *i.e*., a subject having a pathology requiring the treatment of the drug molecule. Implanting is effected by surgically or minimally invasively inserting the device body within a human body, e.g., subcutaneously, subdermally, intramuscularly, intraperitoneally, intra brain, and the like.

As used herein, the term "drug" refers to any substance which can be used to trigger, enhance, suppress, control or regulate any biological process (e.g., cell proliferation, differentiation, expansion, apoptosis, secretion, absorption, transmission and the like). For example, such a drug can be a chemical, an organic molecule, a biological moiety (e.g., which is made of nucleic acids, ribonucleic acids, oligosaccharides, carbohydrates, fatty acids, amino acids) and the like.

For example, a drug molecule capable of treating a heart disease is covalently attached to a ligand capable of binding a specific surface (electrical property) of the artificial receptor of the present invention. Then, the artificial receptor of the present invention is implanted in the subject. For example, the artificial receptor (*i.e.*, the electrode) can be subcutaneously implanted near the heart, similar to a heart pacemaker. Following implantation, the drug molecule is intravenously or orally administered to the subject. However, due to the specific electrical properties configured on the electrode of the artificial receptor of the present invention, the drug molecule preferably binds the implanted electrode. A release of the drug molecule is further effected by modifying the unique surface electrical property of the artificial receptor. As a result, the drug molecule is released *in situ* (*i.e*., at the site to be treated) and is thus far more efficient in treating the pathology.

The device preferably includes an internal power source and a micro receiver and an acoustic transducer. Modification of the unique surface electrical property can be effected essentially as described in U.S. Pat. No. 6,628,989 to Penner et al., by transmission of one or more external acoustic energy waves or signals from an external source into the subject's body, e.g., generally towards the location of the implanted device until the signal is received by the acoustic transducer. Upon excitation by the acoustic wave(s), the acoustic transducer produces an electrical output configured to generate a voltage capable of modifying the electrical properties of the electrodes in the artificial receptor. It will be appreciated that other types of activation of EM energy can also be used, such as RF, etc.

In one possible application, a variation of the present embodiments is used to provide controlled drug release. A quantity of the drug to be released is held in a reservoir, and in the meantime a molecule for which the artificial receptor has an affinity is released into the bloodstream. The molecule that is released has a magnetic particle attached thereto, thus enabling the attachment of the particle to be sensed at the device. The molecule with the magnetic particle reaches the artificial receptor and binds thereto. The magnetic particle is detected via its magnetic field. Detection of the magnetic particle triggers release of the drug. The reservoirs can be placed with the devices deep inside the body at the points where drug administration is required. The particles can then be systematically administered to control release of the drug at the device. The particles can be used to ensure that a given quantity of the drug is released using timing based say on the half-life of the drug within the body. An example is provided in Figure 11.

In a further possible application, an endogenous ligand binds to the artificial receptor. Binding of the ligand affects the electric field of the device temporarily and may cause a temporary signal spike which may be detected following suitable noise reduction. The ligand may be selected to be representative of biological activity that it is desired to monitor. For example the ligand may be an antibody, and the presence or level of too many of the antibodies may indicate a certain condition. The condition may be treatable with a given drug which can be part of a controllable release feature as before. An example is provided in Figure 12.

Thus detection of binding of the given ligand may be used to trigger controlled drug release as in the previous application. Assuming that the drug operates to calm down the condition and reduce the number of the given ligands, a system of negative feedback in fact becomes available for the condition.

It will be appreciated that the artificial receptor of the present invention can be also used to identify small molecules capable of mimicking large molecules (e.g., proteins) or cells.

Thus, according to yet an additional aspect of the present invention, there is provided a method of identifying a small molecule capable of mimicking a binding function of a ligand.

As used herein, the phrase "binding function" refers to the result of binding of a ligand (e.g., an antibody or any other biological moiety) to the artificial receptor of the present invention.

The method is effected by (a) exposing the ligand to at least one electrode configured capable of a unique surface electrical property enabling a specific binding of the ligand thereto, thereby identifying at least one electrode capable of specifically binding the ligand; and (b) identifying a small molecule of a plurality of small molecules capable of binding the at least one electrode being identified as capable of specifically binding the ligand, the small molecule being capable of mimicking the binding function of the ligand.

The method is based on the specific binding of a ligand which is any biological moiety as described hereinabove (e.g., an antibody) to the artificial receptor of the present invention (e.g., at least one electrode as described hereinabove). Following the identification of a specific surface or region in the artificial receptor which bind the ligand, the same region or surface is exposed to a plurality of small molecules, e.g., peptides, using for example, a phage display peptide library as described in Example 3 of the Examples section which follows. Thus, a small molecule which specifically binds to the same surface or region as the ligand is capable of mimicking the ligand binding function.

Thus the teachings of the method according to this aspect of the present invention can be used to identify the binding domains responsible for interactions between antibodies to foreign intruding antigen molecules, hormones and receptors, proteins capable of binding specific receptors on cancer cells and the like.

For example, a peptide, identified as described hereinabove, which is capable of mimicking the binding of a large protein (e.g., a hormone) to a receptor present on a cancer cell (e.g., estrogen receptor present in breast cancer cells) can be used as a targeting vehicle to deliver a drug molecule to the cancer cells, thus preventing and/or treating the subject having cancer. Briefly, a drug molecule (e.g., an agent capable of preventing cell division) is covalently attached to the peptide mimicking the large protein and is administering to the subject (e.g., using intravenous administration). The peptide is specifically recognized by the receptor on the cancer cells and the drug molecule enters the cancer cell and prevents cell proliferation.

Similarly, peptides mimicking the binding of proteins such as growth factors and cytokines can be used as agents for controlling the proliferation and/or differentiation of cells *in vivo* (for determination of factor effects), *ex vivo* (for preparation of cells prior to transplantation in a body) and even *in vivo* (for direct effect in the body).

It will be appreciated that peptides mimicking proteins which are capable of binding specific cell receptors, such as receptors on heart cells, can be used in facilitating the homing of stem cells to specific cells expressing such receptors. Briefly, the peptides are expressed on *ex vivo* expanded pluripotent or partially committed progenitor cells using an expression vector and known molecular biology techniques. The *ex vivo* expanded cells are then transplanted in a recipient subject and the peptide, which is displayed on the transplanted cell membrane as part of a cellular receptor is likely to home to heart cells which present the specific electric field generated by the artificial receptor used in the identification of such a peptide.

Once specific antibodies or peptides with appropriate specificity and affinity are generated, such antibodies or peptides can be engineered to contain a fused moiety that triggers or suppresses a biochemical or biological reaction.

To demonstrate triggering of a biochemical reaction, chimeric proteins are preferably prepared by fusing an enzyme such as peroxidase or alkaline phosphatase to the recombinant antibody and expressing such a construct in E. coli. Alternatively, the antibody is engineered with a specific peptide tag at the C-teminus for site specific biotinylation which enables further manipulation through biotin-streptavidin interaction. To demonstrate activation of a biological process the selected antibodies are fused with antigens capable of triggering different immune responses. Alternatively they are fused with a DNA binding protein and used to affect in-vitro gene expression.

It will be appreciated that the artificial receptor of the present invention can be can be used to characterize a binding site of a ligand. Briefly, such characterization can be achieved by first exposing the ligand to a plurality or an array of electrodes of the artificial receptor of the present invention and isolating and electrically characterizing at least one electrode exhibiting specific binding to the ligand.

As used herein the phrase "electrically characterizing" refers to determining the electric field generated by a surface of the at least one electrode. Such an electric field can be characterized in terms of field strength and/or field shape or spatial distribution on or above the surface (two dimensional or three dimensional).

Altogether, in contrast to a conventional (chemical or biological) receptor, the electronic or artificial receptor of the present invention can be reconfigured in real time to select a desired molecule out of a certain collection. Since each of the antibodies can be fused to an additional molecule having a certain biological function, elution of the bound molecules after rinsing all other molecules may be designed to trigger a desired process corresponding uniquely to the original electrode biasing pattern. Alternatively, binding of selected antibodies to the electrodes removes these molecules from the solution and blocks their corresponding biological processes. The present invention thus takes the interface between manmade electronics and molecular biology a giant leap forward. The teachings of the present invention can be used to facilitate activation or suppression of specific biological pathways based on electronically programmable signals. The implications of such an interface are far reaching. At one end of the spectrum it can be harnessed to electrically control biological processes. For instance, the fused segments may comprise antigens that activate a certain immune response or gene expression. One may envision sensors that probe several biological parameters and feed these signals into electronics that processes the data and activates the required biological pathway using the proposed artificial receptor. The ease of computation by electronic logic provides unparalleled flexibility compared with pure biology alternatives. Moreover, since electronic signals can be generated in the electrodes in response to an electronically transmitted signal, the proposed approach also facilitates remote activation of biological processes. At the other end of the spectrum the scheme provides a promising strategy for closing feedback loops from electronic functionality of biologically constructed nanoelectronics to the construction process itself. To clarify the latter point consider the efforts in recent years to harness molecular biology for the self-assembly of molecular scale electronics. DNA molecules and related proteins were used to scaffold the assembly of a functional transistor made out of non-biological ingredients. Once the transistor was made, there was no way to test its electronic functionality in situ and feedback to the biological assembly process. Without such feedback it is hard to imagine self-assembly of significantly more complex circuits. Feedback from electronic functionality to the assembly process is hence the bottleneck for large scale integration.

The presently preferred embodiments add a new dimension to the interface between nano-electronics and biology and may dramatically affect both fields. The electrode device of Figures 2a-d embodies an artificial receptor whose target antibodies can be changed in real time. Such flexibility is unmatched in biology. Further fascinating possibilities are associated with the potential of electrical control over the bound antibody activity and the extent of control over the bound protein activity which can be achieved by bias of the electrical properties of the artificial receptor of the present invention.

Finally, in the spirit of previous experiments on harnessing molecular biology to the self-assembly of molecular scale electronics, a direct recognition of biased nanoelectronic components by the biological moieties provides unparalleled flexibility and adds a revolutionary new concept to the tools developed thus far.

It is expected that during the life of this patent many relevant switching devices and field carrying materials will be developed and the scopes of the corresponding terms herein are intended to includes all such new technologies *a priori.*

As used herein the term "about" refers to ± 10 %.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., Ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (Eds.) "Genome Analysis: A Laboratory Manual Series", Vols, 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., Ed. (1994); "Culture of Animal Cells - A Manual of Basic Technique" by Freshney, Wiley-Liss, N. Y. (1994), Third Edition; "Current Protocols in Immunology" Volumes I-III Coligan J. E., Ed. (1994); Stites et al. (Eds.), "Basic and Clinical Immunology" (8th Edition), Appleton & Lange, Norwalk, CT (1994); Mishell and Shiigi (Eds.), "Selected Methods in Cellular Immunology", W. H. Freeman and Co., New York (1980); available immunoassays arc extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., Ed. (1984); "Nucleic Acid Hybridization" Hames, B. D., and Higgins S. J., Eds. (1985); "Transcription and Translation" Hames, B. D., and Higgins S. J., Eds. (1984); "Animal Cell Culture" Freshney, R. I., Ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader. All the information contained therein is incorporated herein by reference.

### EXAMPLE 1

### SELECTION OF PHAGE-DISPLAY ANTIBODIES WHICH SPECIFICALLY BIND TO SEMICONDUCTOR SURFACES

To select for specific binders of an electrostatic field, a recombinant phage display scFV antibody library was used for panning on specific semiconductor surfaces, as follows.

### Materials and Experimental Methods

***Semiconductor crystals -*** Galium Arsenite (GaAs) (American Xtal technology Cat. No. 5129327) or silicone (Wacker chmitronic GMBH) crystals, were cut through two planes: The 100 plane which is parallel to one of the surface plane of the crystal and the 111 plane of the crystal. The semiconductor crystals used were: silicon (100), GaAs (100), and GaAs (111A). The crystals at the 100 plane were round with a diameter of about 5 cm; The crystals at the 111 plane were triangles. The width of all crystal wafers was 0.5 mm, regardless of its plane.

For panning (*i.e*., selecting for antibodies which bind the semiconductor surface), each crystal disc of the 100 crystals was cut into a square of 1 x 1 cm² and each of the 111 crystals was cut to a 1 cm long triangle. The four rounds of panning were performed in Eppendorf tubes using a 360 °C tube rotator (Labquake Labotal).

For the ELISA assay, the crystal wafers were cut into small squares which fit the 96-well ELISA plate.

***Selection of antibody molecules against specific crystal facets of semiconductor -*** A human phage display library, Ronit 1, was used for the selection. The library was constructed by Ronit Azriel and Itai Benhar from the faculty of life sciences at Tel Aviv University, Israel (kindly provided by Prof. Itai Benhar) and contains 1 x 10⁹ independent scFv clones. The library is composed of different human synthetic single chain Fv fragments, with variable VH and VL genes in the CDR3 region and is known to generate specific binders to a host of biological targets. The RONIT 1 library consists of a principal in which *in vivo* formed complementarity determining regions were shuffled combinatorially onto germline-derived human variable-region frameworks. The arraying of library-derived scFvs is facilitated by a unique display/expression system, where scFvs are expressed as fusion proteins with a cellulose-binding domain. This library was screened against a number of peptides, proteins, and peptide-protein complexes and yielded antibody fragments exhibiting dissociation constants in the low nanomolar range.

In principle, the selection protocol was similar to that used for selecting biological targets (e.g., as described for the antibodies against the scMHC-peptide complexes) as detailed in Denkberg G., et al., 2002; The J. of Immunology 169: 4399-4407, except that some optimization was performed to adjust for the semiconductor surfaces.

### Optimization of the scanning conditions for the semiconductor surfaces

***Crystal specific pH elution conditions -*** For the GaAs (100) and GaAs (111A), elution was superior in basic conditions (e.g., TEA at pH 12) rather than acidic conditions. On the other hand, for the silicone crystals (both 100 and 111), elution was performed under acidic conditions (e.g., Glycine-HCl, at pH 2.2).

***Blocking of non-specific binding to semiconductor surfaces -*** GaAs substrates (*i.e*., phages containing antibodies) showed extensive non-specific binding of the phages to the substrate via their coat proteins. Although the binding energy per protein was small, their excess number when compared to the number of expressed antibodies dominated the panning assay. To reduce the non-specific binding, the semiconductor surfaces were blocked for 1 hour with 1 % milk, followed by 6 washes with TBS.

***Depletion cycles -*** In order to develop specific binders for specific semiconductor surfaces, the phage display library was exposed for one type of surface [e.g., GaAs (111)] prior to being exposed to another surface [e.g., GaAs (100)], thus the clones capable of binding the first surface [GaAs (111)] were depleted from the library. It is worth mentioning that no wash step was performed between the depletion step [e.g., panning on the GaAs (111)] and the following panning step [e.g., panning on the GaAs (100)].

***Selection of Phage-Antibodies- naïve library -*** Bound phages were eluted from surfaces using TEA at pH 12 [for GaAs (100) and GaAs (111A)] or Glycine-HCl, at pH 2.2 (for the silicone crystals). Eluted phages were used to infect TG1 E. Coli cells (at OD_{600 nm} = 0.5) for 60 minutes at 37 °C, following which the infected bacteria was plated on 2YT plates containing 100 µg/ml ampicilin (2YT/A/G) and 1 % glucose (Sigma G5767).

***Each paning step -*** Colonies from bound phages were collected from the 2YT/A/G plates and diluted 1:100 in 25 ml of 2YT/A/G medium. Cells were grown to O.D_{600 nm} = 0.5 and M13KO7 helper phage [5 x 10¹¹ colony forming unit (cfu)] was added to 25 ml of the culture. After incubation for 60 minutes at 37 °C, the cells were centrifuged, resuspended in 25 ml of 2YT/Ampicillin (100 µg/ml)/Kanamycin (50 µg/ml) and grown overnight at 30°C. Phages were collected from culture supernatants and purified for the next round of panning by PEG precipitation. This procedure repeated 4 times. The diversity of the selected antibodies was determined by RNA fingerprinting.

***Rescue of phages from individual colonies -*** To rescue phage, single ampicillin-resistant colonies resulting from infection of TG1 bacteria with phages, were inoculated into 100 µl of 2xTY containing 100 µg/ml Ampicillin and 1 % glucose (2 x TY-Amp-glucose) in 96-well plates and grown overnight at 37 °C. Five to twenty microlitters of the overnight cultures were inoculated into 150 µl fresh 2 x TY-Amp, to an OD₆₀₀ nm = 0.5 (about 1.5-2 hours). The M13KO7 helper phage [25 µl of 2 x TY-Amp-glucose containing 10⁹ plaque forming unit (p.f.u.)] was then added to each well, and the plate was incubated without agitation for 60 minutes at 37 °C. The plate was then shaken at 37 °C for one hour after which the cells were pelletted at 4000 rpm for 15 minutes. The cells were then resuspended in 200 µl 2 x TY containing 100 µg/ml Amp and 25 µg/ml Kan (2 x TY-Amp-Kan) and grown overnight at 30 °C, The cells were pelletted as above, and the supernatants containing the phages were tested for their binding to the semiconductor surface using the ELISA assay.

***ELISA conditions on semiconductor surfaces -*** For ELISA assay, the semiconductor surfaces were cut into small squares to fit the 96-well ELISA plate, incubated overnight with 1 % milk in TBS, and washed 6 times with TBS (200 µl each wash). In each well, about 60 µl of a phage suspension (isolated following 4 panning as described above) was mixed with TBS to reach a final volume of 150 µl/well. The reaction was incubated for one hour at room temperature while shaking, following which the fluid was aspirated, and the wells including the semiconductor surfaces were washed 6 times in TBS containing 0.1 % of Tween®-20. To reveal antibody-bound surfaces, an HRP/anti M13 monoclonal antibody conjugate (Amersham 27-9421-01) was added (0.125 µl per well), incubated for 1 hour in the presence of 1 % milk, following which the wells were washed 6 times with TBS and the TNB One Step Substrate (S-159985 DAKO Cytomation, Denmark) was added. The ELISA reactions were read at 450 nm. The DNA of positive phage clones (*i.e.*, phage clones displaying OD values of 0.750 or higher in the ELISA assay) was further characterized by *Bst*NI restriction analysis and/or sequencing.

### Experimental Results

***Panning of phage display antibodies on semiconductor surfaces -*** Phage display antibodies were selected for their binding capacity to the semiconductor surfaces GaAs (100), GaAs (111A) and silicone (100). As is shown in Figure 9, phage display antibodies selected after the fourth panning on the GaAs (111) exhibited similar binding efficacy on both GaAs (111) and GaAs (100) (Figure 9, two left columns). To increase the selectivity of binding to a specific surface, the phage display antibodies were subjected to a depletion panning on a different semiconductor surface prior to panning on the specific surface. Table 1, hereinbelow, summarizes the panning results.
***Table 1***

**Table 1: The No. of phage clones eluted at each panning round is presented. Note the effect of a depletion panning on the selectivity of binding to a specific surface.**

| ***Enrichment of binders to GaAs (100), GaAs (111A) and Silicon vs. panning round*** | | | | | |
|---|---|---|---|---|---|
| ***Panning round*** | ***GaAs 100 (phage*/*ml)*** | | ***GaAs 111A (phage*/*ml)*** | | ***Silicon (phage*/*ml)*** |
| | ***With depletion to GaAs (IIIA)*** | ***Without depletion to GaAs (111A)*** | ***With depletion to GaAs (100)*** | ***Without depletion to GaAs (100)*** | |
| 1 | 3*10⁵ | 3*10⁵ | 3*10⁴ | 3*10⁴ | 6.*10⁴ |
| 2 | 1*10⁵ | 6*10³ | 3*10⁶ | 1*10⁴ | 7.*10⁶ |
| 3 | 1*10⁷ | 2*10⁶ | 9*10⁶ | 1*10⁶ | 1.*10⁸ |

***Depletion cycles on a certain semiconductor facet increased binding affinity of phage display antibodies towards the other facet -*** As is shown in Table 1, hereinabove, at the second and third rounds of panning, the selectivity of binding to the GaAs (111A) surface increased by about an order of magnitude following depletion steps over the GaAs (100) surface. Similarly, at the second and third rounds of panning on the GaAs (100) surface, the selectivity of binding to the GaAs (100) surface increased by an order of magnitude following depletion steps on the GaAs (111A) surface. In addition, when the pool of the phage display antibodies was depleted by panning on cycle 2 and 3 on the GaAs (100) surface, the phage display antibodies selected following the forth panning on the GaAs (111) exhibited a significant higher binding efficacy (by almost two orders of magnitudes) towards the GaAs (111) surface than towards the GaAs (100) surface (Figure 9, two right columns).

***ELISA analysis confirmed selectivity of phage display antibodies to the semiconductor surfaces -*** After 4 rounds of panning and 2 rounds of depletion, several clones were subject to an ELISA assay. About 60 % of the clones showed markedly enhanced binding to their target compared to the control group. The readings are summarized in Table 2, hereinbelow. Clones E1, F1 and C7 were selective to GaAs (111A), clone F10 was selective to GaAs (100), and clone B7 binds both surfaces.
***Table 2***

**Table 2: Shown are ELISA readings measured at 450 nm in 96-well plates.**

| ***ELISA analysis of selected phage display antibodies*** | | |
|---|---|---|
| Clone | GaAS 111A | GaAS 100 |
| E1 | 0.984 | 0.204 |
| F1 | 0.849 | 0.234 |
| F10 | 0.142 | 0.890 |
| B7 | 1.236 | 0.942 |
| C7 | 0.826 | 0.206 |

To further confirm the specificity of binding to the surface, the DNA of all five clones was sequenced for the V_{H} CDR3 and V_{L} CDR3 genes. Tables 3 and 4, hereinbelow, present the sequences of the V_{H} and V_{L} CDR3 genes, respectively, of the isolated clones.

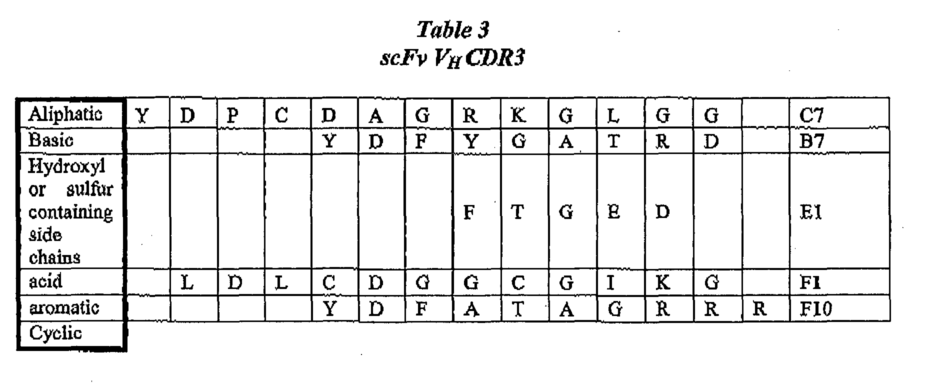

As is shown in Table 3, hereinabove, no definite consensus sequence in the V_{H} area of the CDR3 region was found. However, the percentage of hydrophobic amino acids was considerably higher than their abundance in the library.

**Table 4**

| ***scFv V_{L} CDR3*** | | | | | | | | | |
|---|---|---|---|---|---|---|---|---|---|
| 1 | 2 | 3 | 4 | 5 | 6 | 7 | 8 | 9 | |
| | | P | S | N | G | Y | Q | Q | C7 |
| R | N | G | S | S | D | R | S | N | B7 |
| A | D | L | R | S | D | S | T | Q | E1 |
| A | S | L | R | S | D | Y | S | Q | F1 |
| | | T | D | S | D | W | A | Q | F10 |

On the other hand, a clear consensus sequence was observed in the V_{L} area of CDR 4 region (columns 3, 5, 6, and 9). These results therefore demonstrate that the selectivity towards the GaAs semiconductor surface is conferred by the hydrophobic nature of the heavy CDR and specific amino acid consensus in the light chain CDR.

Altogether, these unexpected results demonstrate that phage display antibodies which specifically bind to semiconductor surfaces (*i.e*., binders) can be isolated, preferably following one or two depletion cycles. Thus, specific binders to the GaAs (111A) were isolated following 2 depletions on the GaAs (100) surface.

### EXAMPLE 2

### EXPRESSION OF PHAGE-DISPLAY ANTIBODIES WHICH BIND TO SEMICONDUCTOR SURFACES

### Materials and Experimental Methods

***Expression and purification of soluble scFv antibodies* -** The soFV gene is rescued from the phage clone by PCR and is then subcloned into the phagemid vector pCANTAB6 by using the *SfiI-NotI* cloning sites. A Myc and hoxahistidine tags are fused to the C-terminus of the scFv gene. The soFv antibody is expressed in BL21 λDE3 cells and purified from the periplasmic fraction by metal-ion affinity chromatography:
Soluble ScFv are purified from the periplasmic fraction of BL21 cells using the hexahistidine tag. An overnight starter culture of Fab specific clones is grown at 30 °C. Bacterial cells are diluted 1:100 into 500 ml of 2YT/A/G, grown to OD₆₀₀ₙₘ = 0.8-1.0 and induced to express the recombinant ScFv antibody by the addition of 1 mM IPTG for 3 hours at 30 °C. The bacterial cells are centrifuged and the pellet is resuspended in 5 ml of a B-PER solution (Pierce) to release periplasmatic content. After 30 minutes of rotated incubation at room temperature, the solution is centrifuged (15000 rpm, 15 minutes) and the supernatant is incubated with 0.5 ml of pre-washed TALON beads suspension (Clontech) for 45 minutes at room temperature. The solution is applied onto a BioRad (Hercules, CA) disposable column, and after sedimentation the beads are washed three times with 10 ml of PBS/0.1 % Tween® 20 (pH 8.0). The bound ScFvs are eluted using 0.5 ml of 100 mM Imidazole in PBS. To remove residual imidazole, the eluted scFv are dialyzed twice against PBS (overnight, 4 °C). The homogeneity and purity of the purified ScFv / Fabs is determined by analysis on non-reduced and reduced SDS-PAGE.

### EXAMPLE 3

### SELECTION OF PHAGE DISPLAY PEPTIDES WHICH BIND TO SEMICONDUCTOR SURFACES

Prior art studies applied specific crystal facets of metals, oxides, minerals, and semiconductors for the selective binding of peptides. The present inventors screen a phage display peptide library for selective binders of the GaAs (100) and GaAs (111A) surfaces, as follows.

### Materials and Experimental Methods

***Panning of semiconductor surfaces with a phage display peptide library** -* The Ph.D.-7 Phage Display Peptide Library Kit (New England Bio Labs Inc., Beverly, MA, USA) was used to screen for positive phages displaying peptides capable of binding to the following semiconductor surfaces: GaAs (100), and GaAs (111A). The panning protocol was exactly as recommended by the kit's manufacturer except that panning was performed on crystal surfaces as described in Example 1, hereinabove.

### Experimental Results

***Selection of peptides against specific cystal facets of semiconductors -*** The New England Biolabs random peptide library is an exhaustive collection of linear heptapeptide (1.28 x 10⁹ different peptides). The randomized peptide sequences are expressed at the N-terminus of the minor coat protein pIII, resulting in a valency of 5 copies of the displayed peptide per virion.

The GaAs (100) and GaAs (111A) were screened using the Ph.D-7 phage display peptide library using 4 rounds of panning. Figure 10 depicts the enrichment of binders to GaAs (100) and GaAs (111A) vs. panning round.

***Clones selected following panning on the GaAs 100 surface -*** Clones were sequenced after each panning cycle on the GaAs (100) crystal. As seen in Tables 5, 6, and 7, hereinbelow, the third panning round (Table 7) produced 10 identical clones and another subset of two identical clones. Hence, the binding of other peptides to GaAs (100) must be significantly less efficient.

**Table 5**

| ***Clones from panning 1 on GaAs 100 surface*** | | | | | | | | | | | | | |
|---|---|---|---|---|---|---|---|---|---|---|---|---|---|
| G | G | G | S | S | S | T | S | H | F | S | H | S | Out1_100-4_02 |
| G | G | G | M | Q | T | Y | T | N | S | S | H | S | Out1_100-5_04 |

**Table 6**

| ***Clones from panning 2 on GaAs 100 surface*** | | | | | | | | | | | | | | | |
|---|---|---|---|---|---|---|---|---|---|---|---|---|---|---|---|
| G | G | G | R | S | V | Q | L | T | L | S | H | S | | | Out2_100-6_09 |
| G | G | G | S | R | A | Q | T | Y | A | S | H | S | | | Out2_100-1_16 |
| | | G | G | G | P | S | E | A | L | H | W | S | H | S | Out2_100-3_03 |
| | G | G | G | F | L | Q | S | T | I | H | S | H | S | | Out2_100-2_01 |
| | G | G | G | R | L | N | H | E | H | S | S | H | S | | Out2_100-5_07 |

**Table 7**

| ***Clones from panning 3 on GaAs 100 surface*** | | | | | | | | | | | | | | |
|---|---|---|---|---|---|---|---|---|---|---|---|---|---|---|
| G | G | G | Y | T | Y | M | A | P | L | S | H | S | | 10 identical clones |
| | G | G | G | S | R | K | L | P | M | Y | S | H | S | Out3_100-4_10 |
| | G | G | S | S | R | K | L | P | M | Y | S | H | S | Out3_100-1_04 |
| G | G | G | D | P | W | E | I | T | T | S | H | S | | Out3_100-3_08 |
| | G | G | G | L | L | T | T | T | T | G | S | H | S | Out3_100-6_14 |

***Clones selected following panning on the GaAs 111A surface** -* Clones were sequenced after each panning cycle on the GaAs (111) crystal. As seen in Tables 7 and 8, hereinbelow, the sequences selected to GaAs (111) show a clear consensus sequence.

**Table 8**

| ***Clones from panning 3 on GaAs 111 surface*** | | | | | | | | | | | | | | | | |
|---|---|---|---|---|---|---|---|---|---|---|---|---|---|---|---|---|
| G | G | G | L | P | P | P | T | Y | T | S | H | S | | | | Out3_111_1 |
| G | G | G | D | S | I | P | S | H | V | S | H | S | | | | Out3_111_2 |
| | | | G | G | G | P | S | S | E | Y | Q | W | S | H | S | Out3_111_3 |
| | | | V | S | L | P | S | V | A | | | | | | | Out3_111_4 |
| | G | G | G | T | I | I | T | H | H | Q | S | H | S | | | Out3_111_5 |
| | G | G | G | T | I | I | T | H | H | Q | S | H | S | | | Out3_111_6 |
| | G | G | G | T | I | I | T | H | H | Q | S | H | S | | | Out3_111_7 |
| | G | G | G | I | P | W | S | H | F | D | S | H | S | | | Out3_111_8 |
| | G | G | G | I | P | W | S | H | P | D | S | H | S | | | Out3_111_9 |

**Table 9**

| ***Clones from panning 4 on GaAs 111 surface*** | | | | | | | | | | | | | | |
|---|---|---|---|---|---|---|---|---|---|---|---|---|---|---|
| | G | G | G | P | L | H | R | P | T | H | S | H | S | Out4_111A-1 |
| | G | G | G | T | A | W | L | P | T | W | S | H | S | Out4_111A-2 |
| | G | G | G | R | Q | L | E | L | Q | A | S | H | S | Out4_111A-3 |
| G | G | G | R | F | D | H | G | A | T | S | H | S | | Out4_111A-4 |
| | G | G | G | A | M | P | Q | R | P | L | S | H | S | Out4_111A-5 |

These results clearly demonstrate that both the GaAs 111 and GaAs 100 surfaces were capable of selectively bind specific phage display peptides sharing a consensus sequence.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

## Claims

1. An electrode comprising:
a surface having a variable and configurable electrostatic field over an extent of said surface, and
at least one substance attached to surface and being capable of modifying at least one of a hydrophobic property, charged state, hydrophilic property, and said variable electrostatic field to provide specific binding for a target biological moiety.

2. The electrode of claim 1, wherein said at least one substance comprises a piezoelectric substance configurable to alter said electrostatic field.

3. The electrode of claim 1 or claim 2, wherein said surface is a surface of a metal-insulator superlattice comprising metal and insulative layers.

4. The electrode of any of claims 1 - 3 wherein said surface comprises switchable wiring for controllably allowing changes to said electrostatic field.

5. The electrode of any of claims 1 - 4, wherein
said surface comprises switchable electrical conductive tracks, said conductive tracks being switchable to configure said electrostatic field about said surface to provide said specific binding for said target biological moiety.

6. The electrode of any of claims 1 - 5, comprising:
a plurality of semiconductor nanocrystals extending over said surface, said nanocrystals comprising P-N junctions,
wherein at least some of said nanocrystals is independently electrifiable, such as to produce said electrostatic field.

7. An array comprising a plurality of addressable locations each including an electrode according to any of claims 1 - 6.

8. The electrode or array of any of claims 1 - 7, wherein the electrode further comprises a structure having a plurality of layers, wherein said surface being in a plane substantially cross-sectional to said plurality of layers, and
at least some of said layers being independently electrifiable, such as to produce said electrostatic field.

9. A kit for typing ligands comprising:
the electrode or array of any of claims 1 - 8, wherein said electrostatic field is configurable for specifically binding the ligand, and
reagents for qualifying binding of the ligands to said electrodeelectrode.

10. A method, comprising:
exposing a ligand to an electrode which comprises a surface having a variable and configurable electrostatic field over an extent of said surface, and at least one substance attached to surface and being capable of modifying at least one of a hydrophobic property, charged state, hydrophilic property, and said variable electrostatic field to provide specific binding for said ligand, thereby identifying specificity of said surface or portion thereof to said ligand.

11. The method of claim 10, further comprising identifying a small molecule of a plurality of small molecules capable of binding said electrode, said small molecule being capable of mimicking the binding function of the ligand.

12. The method of claim 11, wherein said ligand is present in a mixed population of ligands, and wherein said exposure results in isolation of said ligand from the mixed population of ligands.

13. A device for controllable delivery of a drug molecule to a tissue, comprising an electrode according to claim 1, wherein said electrostatic field enables a specific binding of a ligand thereto; said ligand being attached to the drug, said electrostatic field being modifiable by a switching unit to control a binding or a release of said ligand and thereby to controllably deliver the drug molecule to the tissue.

14. The electrode, the array, the kit, the method or the device of any of claims 1 - 13, wherein said surface comprises a plurality of regions over said extent, each region having a predetermined electrostatic field strength.

15. The electrode of claim 1, wherein said at least one substance is further capable of modifying interaction of said surface with said target biological moiety, said interaction being selected from the group consisting of hydrogen bonding and van der walls interaction.

16. The electrode of claim 1, wherein said at least one substance is an organic substance.

17. The electrode of claim 16, wherein said organic substance is selected from the group consisting hydroquinone and rotaxane.

18. The electrode of claim 1, wherein said at least one substance is a biological substance.

19. The electrode of claim 18, wherein said biological substance is a peptide, a protein, a lipid, a carbohydrate and/or nucleic acids.

20. The electrode of claim 1, wherein said at least one substance is a Ferroelectric substance, and/or a Pyroelectric substance.

21. The electrode, the array, the kit, the method or the device of claim 14, wherein said regions are on the order of magnitude of nanometer, Angstrom and/or tens of nanometers.

22. The electrode, the array, the kit, the method or the device of claim 14, wherein said regions are in the order of magnitude of five to ten lattice constants.

23. The electrode, the array, the kit, the method or the device of claim 14, wherein variable electrical fields are applied to said regions.

24. The electrode, the array, the kit, the method or the device of claim 14, comprising a laminate including a plurality of independently electrifiable layers, said surface being formed from a cross-section of said laminate such that said regions are defined by said independently electrifiable layers.

25. The electrode, the array, the kit, the method or the device of claim 24, further comprises a selector for selecting a voltage level for each of said independently electrifiable layers, thereby to allow said electrostatic field to be varied to provide specific binding to different target moieties.

26. The electrode, the array, the kit, the method or the device of claim 21, wherein said surface comprises an electronically controllable hydrophobic coating, thereby to allow controllable hydrophobic properties per said independently electrifiable layer.

27. The electrode of claim 3, wherein said insulative layers comprise one member of the group consisting of a metal oxide, a semiconductor and a ceramic.

28. The electrode of any of claims 1-8, 13-17 and 27, wherein said electrostatic field is controllable by means of carbon nanotubes and/or semiconductor nanowires.

29. The electrode of claim 28, further comprising a selector for selecting a voltage level for each of said carbon nanotubes and/or semiconductor nanowires, thereby to allow said electrostatic field to be varied to provide specific binding to different targeted moieties.

30. The electrode of claim 8, further comprising a switching unit for switching said layers such as to configure said electrostatic field.

31. The electrode of claims 4 and/or 8, further comprising a covering layer over said surface.

32. The electrode of claim 8, wherein said plurality of layers have a transverse direction and a longitudinal direction at said surface and wherein said surface has a transverse direction and a longitudinal direction and wherein said layers are aligned about said surface such that said layer transverse direction lies along said surface longitudinal direction.

33. The electrode of claim 4, wherein said semiconductor nanocrystals are remotely electrifiable via incident radiation.

34. The electrode of claim 4, further comprises a substance storage and release mechanism associated with said surface, such that a given change in said electrostatic field is operable to affect said storage and release mechanism to effect release of a substance stored therein.

## Patentansprüche

1. Elektrode, umfassend:
eine Fläche mit einem variablen und konfigurierbaren elektrostatischen Feld über einem Bereich der Fläche, und
mindestens eine Substanz, die an der Fläche befestigt ist und dazu in der Lage ist, mindestens eines einer hydrophoben Eigenschaft, eines Ladezustands, einer hydrophilen Eigenschaft und des variablen elektrostatischen Felds zu modifizieren, um eine spezifische Bindung für einen biologischen Zielteil bereitzustellen.

2. Elektrode nach Anspruch 1, wobei die mindestens eine Substanz eine piezoelektrische Substanz umfasst, die konfiguriert werden kann, um das elektrostatische Feld zu verändern.

3. Elektrode nach Anspruch 1 oder Anspruch 2, wobei die Fläche eine Fläche aus einem Metall-Isolator-Supergitter ist, umfassend Metall- und isolierende Schichten.

4. Elektrode nach einem der Ansprüche 1 - 3, wobei die Fläche eine schaltbare Verkabelung umfasst, um kontrollierbar Änderungen an dem elektrostatischen Feld zu ermöglichen.

5. Elektrode nach einem der Ansprüche 1 - 4, wobei
die Fläche schaltbare elektrische leitfähige Spuren umfasst,
wobei die leitfähigen Spuren schaltbar sind, um das elektrostatische Feld um die Fläche zu konfigurieren, um die spezifische Bindung für den biologischen Zielteil bereitzustellen.

6. Elektrode nach einem der Ansprüche 1 - 5, umfassend:
eine Vielzahl von Halbleiter-Nanokristallen, die sich über die Fläche erstrecken, wobei die Nanokristalle PN-Übergänge umfassen,
wobei mindestens einige der Nanokristalle unabhängig voneinander elektrifizierbar sind, um das elektrostatische Feld zu erzeugen.

7. Anordnung, umfassend eine Vielzahl von adressierbaren Stellen, wobei jede eine Elektrode nach einem der Ansprüche 1 - 6 umfasst.

8. Elektrode oder Anordnung nach einem der Ansprüche 1 - 7, wobei die Elektrode weiter eine Struktur aufweist, umfassend eine Vielzahl von Schichten, wobei die Fläche eine Ebene ist, die im Wesentlichen einen Querschnitt mit Bezug auf die Vielzahl von Schichten darstellt, und
wobei mindestens einige der Schichten unabhängig voneinander elektrifizierbar sind, um das elektrostatische Feld zu erzeugen.

9. Satz zum Typisieren von Liganden, umfassend:
die Elektrode oder die Anordnung nach einem der Ansprüche 1 - 8, wobei das elektrostatische Feld konfiguriert werden kann, um den Liganden spezifisch zu binden, und
Reagenzien, um die Bindung der Liganden an die Elektrode zu qualifizieren.

10. Verfahren, umfassend:
Aussetzung des Liganden an eine Elektrode, die eine Fläche mit einem variablen und konfigurierbaren elektrostatischen Feld über einem Bereich der Fläche umfasst, und mindestens eine Substanz die an der Fläche befestigt ist und dazu in der Lage ist, mindestens eines einer hydrophoben Eigenschaft, eines Ladezustands, einer hydrophilen Eigenschaft und des variablen elektrostatischen Felds zu modifizieren um eine spezifische Bindung für den Liganden bereitzustellen, wodurch die Spezifizität der Fläche oder des Abschnitts davon zum Liganden identifiziert wird.

11. Verfahren nach Anspruch 10, weiter umfassend die Identifizierung eines kleinen Moleküls einer Vielzahl von kleinen Molekülen, die dazu in der Lage sind, die Elektrode zu binden, wobei das kleine Molekül dazu in der Lage ist, die Bindungsfunktion des Liganden nachzuahmen.

12. Verfahren nach Anspruch 11, wobei der Ligand in einer gemischten Population von Liganden vorhanden ist, und wobei die Aussetzung zu einer Isolierung des Liganden von der gemischten Population von Liganden führt.

13. Vorrichtung zur kontrollierbaren Abgabe eines Arzneimittelmoleküls an ein Gewebe, umfassend eine Elektrode nach Anspruch 1, wobei das elektrostatische Feld eine spezifische Bindung eines Liganden daran ermöglicht; wobei der Ligand an das Arzneimittel befestigt ist, wobei das elektrostatische Feld durch eine Schalteinheit modifiziert werden kann, um eine Bindung oder eine Freigabe des Liganden zu steuern und dadurch das Arzneimittelmolekül kontrollierbar an das Gewebe abzugeben.

14. Elektrode, Anordnung, Satz, Verfahren oder Vorrichtung nach einem der Ansprüche 1 - 13, wobei die Fläche eine Vielzahl von Regionen über dem Bereich umfasst, wobei jede Region eine vorbestimmte elektrostatische Feldstärke aufweist.

15. Elektrode nach Anspruch 1, wobei die mindestens eine Substanz weiter dazu in der Lage ist, die Wechselwirkung der Fläche mit dem biologischen Zielteil zu modifizieren, wobei die Wechselwirkung ausgewählt ist aus der Gruppe bestehend aus Wasserstoffbindung und Van-der-Wals-Wechselwirkung.

16. Elektrode nach Anspruch 1, wobei die mindestens eine Substanz eine organische Substanz ist.

17. Elektrode nach Anspruch 16, wobei die organische Substanz ausgewählt ist aus der Gruppe bestehend aus Hydrochinon und Rotaxan.

18. Elektrode nach Anspruch 1, wobei die mindestens eine Substanz eine biologische Substanz ist.

19. Elektrode nach Anspruch 18, wobei die biologische Substanz ein Peptide, ein Protein, ein Lipid, ein Kohlenhydrat und/oder Nukleinsäuren ist.

20. Elektrode nach Anspruch 1, wobei die mindestens eine Substanz eine ferroelektrische Substanz und/oder eine pyroelektrische Substanz ist.

21. Elektrode, Anordnung, Satz, Verfahren oder Vorrichtung nach Anspruch 14, wobei die Regionen im Größenbereich von Nanometer, Ångström und/oder Dutzenden von Nanometern liegen.

22. Elektrode, Anordnung, Satz, Verfahren oder Vorrichtung nach Anspruch 14, wobei die Regionen im Größenbereich von fünf bis zehn Gitterkonstanten liegen.

23. Elektrode, Anordnung, Satz, Verfahren oder Vorrichtung nach Anspruch 14, wobei variable elektrische Felder auf die Regionen angelegt werden.

24. Elektrode, Anordnung, Satz, Verfahren oder Vorrichtung nach Anspruch 14, umfassend ein Laminat, darin eingeschlossen eine Vielzahl von unabhängig voneinander elektrifizierbaren Schichten, wobei die Fläche aus einem Querschnitt des Laminats gebildet ist, so dass die Regionen durch die unabhängig voneinander elektrifizierbaren Schichten definiert sind.

25. Elektrode, Anordnung, Satz, Verfahren oder Vorrichtung nach Anspruch 24, weiter umfassend einen Selektor, um ein Spannungsniveau für jede der unabhängig voneinander elektrifizierbaren Schichten auszuwählen, und dadurch zu ermöglichen, dass das elektrostatische Feld variiert ist, um eine spezifische Bindung an verschiedene Zielteile bereitzustellen.

26. Elektrode, Anordnung, Satz, Verfahren oder Vorrichtung nach Anspruch 21, wobei die Fläche eine elektronisch steuerbare hydrophobe Beschichtung umfasst, um dadurch steuerbare hydrophobe Eigenschaften durch die unabhängig voneinander elektrifizierbaren Schichten zu ermöglichen.

27. Elektrode nach Anspruch 3, wobei die isolierenden Schichten ein Element der Gruppe umfassen, bestehend aus einem Metalloxid, einem Halbleiter und einer Keramik.

28. Elektrode nach einem der Ansprüche 1 - 8, 13 - 17 und 27, wobei das elektrostatische Feld mit Hilfe von Kohlenstoff-Nanoröhren und/oder Halbleiter-Nanodrähten gesteuert werden kann.

29. Elektrode nach Anspruch 28, umfassend einen Selektor, um ein Spannungsniveau für jedes der Kohlenstoff-Nanoröhren und/oder der Halbleiter-Nanodrähte auszuwählen, und dadurch zu ermöglichen, dass das elektrostatische Feld variiert ist, um eine spezifische Bindung an verschiedene Zielteile bereitzustellen.

30. Elektrode nach Anspruch 8, weiter umfassend eine Schalteinheit, um die Schichten zu schalten, um das elektrostatische Feld zu konfigurieren.

31. Elektrode nach Anspruch 4 und/oder 8, weiter umfassend eine Deckschicht über der Fläche.

32. Elektrode nach Anspruch 8, wobei die Vielzahl von Schichten eine Querrichtung und eine Längsrichtung an der Fläche aufweist, und wobei die Fläche eine Querrichtung und eine Längsrichtung aufweist, und wobei die Schichten um die Fläche ausgefluchtet sind, so dass die Querrichtung der Schicht entlang der Längsrichtung der Fläche liegt.

33. Elektrode nach Anspruch 4, wobei die Halbleiter-Nanokristalle über einfallende Strahlung entfernt elektrifiziert werden können.

34. Elektrode nach Anspruch 4, weiter umfassend einen Mechanismus zur Speicherung und zur Freigabe einer Substanz, der mit der Fläche assoziiert ist, so dass eine bestimmte Änderung des elektrostatischen Felds durchführbar ist, um den Mechanismus zur Speicherung und zur Freigabe zu beeinflussen, um die Freigabe einer Substanz, die darin gespeichert ist, zu bewirken.

## Revendications

1. Électrode comprenant :
une surface ayant un champ électrostatique variable et configurable sur une étendue de ladite surface, et
au moins une substance fixée à la surface et étant capable de modifier au moins l'une d'une propriété hydrophobe, un état chargé, une propriété hydrophile, et ledit champ électrostatique variable pour produire une liaison spécifique pour un fragment biologique cible.

2. Électrode de la revendication 1, dans laquelle ladite au moins une substance comprend une substance piézoélectrique configurable pour modifier ledit champ électrostatique.

3. Électrode de la revendication 1 ou la revendication 2, dans laquelle ladite surface est une surface d'un super-réseau isolant métallique comprenant des couches métalliques et isolantes.

4. Électrode de l'une quelconque des revendications 1 à 3 dans laquelle ladite surface comprend un câblage commutable pour permettre de façon contrôlable des modifications dudit champ électrostatique.

5. Électrode de l'une quelconque des revendications 1 à 4, dans laquelle ladite surface comprend des pistes électriquement conductrices commutables, lesdites pistes conductrices étant commutables pour configurer ledit champ électrostatique autour de ladite surface pour produire ladite liaison spécifique pour ledit fragment biologique cible.

6. Électrode de l'une quelconque des revendications 1 à 5, comprenant :
une pluralité de nanocristaux semi-conducteurs s'étendant sur ladite surface, lesdits nanocristaux comprenant des jonctions P-N,
dans laquelle au moins certains desdits nanocristaux est indépendamment électrifiable, de manière à produire ledit champ électrostatique.

7. Puce comprenant une pluralité d'emplacements adressables comprenant chacun une électrode selon l'une quelconque des revendications 1 à 6.

8. Électrode ou puce de l'une quelconque des revendications 1 à 7, l'électrode comprenant en outre une structure ayant une pluralité de couches, ladite surface étant dans un plan sensiblement en coupe transversale par rapport à ladite pluralité de couches, et
au moins certaines desdites couches étant indépendamment électrifiables, de manière à produire ledit champ électrostatique.

9. Kit pour typer des ligands comprenant :
l'électrode ou puce de l'une quelconque des revendications 1 à 8, ledit champ électrostatique étant configurable pour se lier spécifiquement au ligand, et
des réactifs pour qualifier la liaison des ligands à ladite électrode.

10. Procédé, comprenant :
l'exposition d'un ligand à une électrode qui comprend une surface ayant un champ électrostatique variable et configurable sur une étendue de ladite surface, et au moins une substance fixée à la surface et étant capable de modifier au moins l'une d'une propriété hydrophobe, un état chargé, une propriété hydrophile, et ledit champ électrostatique variable pour produire une liaison spécifique pour ledit ligand, de manière à identifier la spécificité de ladite surface ou partie de celle-ci pour ledit ligand.

11. Procédé de la revendication 10, comprenant en outre l'identification d'une petite molécule d'une pluralité de petites molécules capables de se lier à ladite électrode, ladite petite molécule étant capable d'imiter la fonction de liaison du ligand.

12. Procédé de la revendication 11, dans lequel ledit ligand est présent dans une population mixte de ligands, et dans lequel ladite exposition conduit à l'isolement dudit ligand à partir de la population mixte de ligands.

13. Dispositif pour l'administration contrôlable d'une molécule pharmaceutique à un tissu, comprenant une électrode selon la revendication 1, dans lequel ledit champ électrostatique permet une liaison spécifique d'un ligand à celle-ci ; ledit ligand étant fixé au médicament, ledit champ électrostatique étant modifiable par une unité de commutation pour commander une liaison ou une libération dudit ligand et de manière à administrer de façon contrôlable la molécule pharmaceutique au tissu.

14. Électrode, puce, kit, procédé ou dispositif de l'une quelconque des revendications 1 à 13, dans lesquels ladite surface comprend une pluralité de régions sur ladite étendue, chaque région ayant une intensité de champ électrostatique prédéterminée.

15. Électrode de la revendication 1, dans laquelle ladite au moins une substance est en outre capable de modifier l'interaction de ladite surface avec ledit fragment biologique cible, ladite interaction étant choisie dans le groupe constitué d'une liaison hydrogène et d'une interaction de Van der Walls.

16. Électrode de la revendication 1, dans lequel ladite au moins une substance est une substance organique.

17. Électrode de la revendication 16, dans laquelle ladite substance organique est choisie dans le groupe constitué de l'hydroquinone et du rotaxane.

18. Électrode de la revendication 1, dans laquelle ladite au moins une substance est une substance biologique.

19. Électrode de la revendication 18, dans laquelle ladite substance biologique est un peptide, une protéine, un lipide, un glucide et/ou des acides nucléiques.

20. Électrode de la revendication 1, dans laquelle ladite au moins une substance est une substance ferroélectrique et/ou une substance pyroélectrique.

21. Électrode, puce, kit, procédé ou dispositif de la revendication 14, dans lesquelles lesdites régions sont de l'ordre de grandeur du nanomètre, de l'angström et/ou de dizaines de nanomètres.

22. Électrode, puce, kit, procédé ou dispositif de la revendication 14, dans lesquels lesdites régions sont de l'ordre de grandeur de cinq à dix constantes de réseau.

23. Électrode, puce, kit, procédé ou dispositif de la revendication 14, dans lesquels des champs électrique variables sont appliqués auxdites régions.

24. Électrode, puce, kit, procédé ou dispositif de la revendication 14, comprenant un stratifié comprenant une pluralité de couches indépendamment électrifiables, ladite surface étant formée d'une section transversale dudit stratifié de sorte que lesdites régions soient définies par lesdites couches indépendamment électrifiables.

25. Électrode, puce, kit, procédé ou dispositif de la revendication 24, comprenant en outre un sélecteur pour sélectionner un niveau de tension pour chacune desdites couches indépendamment électrifiables, de manière à permettre que ledit champ électrostatique varie de manière à produire une liaison spécifique aux différents fragments cibles.

26. Électrode, puce, kit, procédé ou dispositif de la revendication 21, dans lesquels ladite surface comprend un revêtement hydrophobe électroniquement contrôlable, de manière à pouvoir obtenir des propriétés hydrophobes contrôlables en ce qui concerne ladite couche indépendamment électrifiable.

27. Électrode de la revendication 3, dans laquelle lesdites couches isolantes comprennent un membre du groupe constitué d'un oxyde de métal, un semi-conducteur et une céramique.

28. Électrode de l'une quelconque des revendications 1 à 8, 13 à 17 et 27, dans laquelle ledit champ électrostatique est contrôlable au moyen de nanotubes de carbone et/ou de nanocâbles semi-conducteurs.

29. Électrode de la revendication 28, comprenant en outre un sélecteur pour sélectionner un niveau de tension pour chacun desdits nanotubes de carbone et/ou nanocâbles semi-conducteurs, de manière à permettre que ledit champ électrostatique varie de manière à produire une liaison spécifique à différents fragments ciblés.

30. Électrode de la revendication 8, comprenant en outre une unité de commutation pour commuter lesdites couches de manière à configurer ledit champ électrostatique.

31. Électrode des revendications 4 et/ou 8, comprenant en outre une couche de couverture sur ladite surface.

32. Électrode de la revendication 8, dans laquelle ladite pluralité de couches ont une direction transversale et une direction longitudinale à ladite surface et dans laquelle ladite surface a une direction transversale et une direction longitudinale et dans laquelle lesdites couches sont alignées autour de ladite surface de sorte que ladite direction transversale de couche soit située le long de ladite direction de surface longitudinale.

33. Électrode de la revendication 4, dans laquelle lesdits nanocristaux semi-conducteurs sont électrifiables à distance via un rayonnement incident.

34. Électrode de la revendication 4, comprenant en outre un mécanisme de stockage et de libération de substance associé à ladite surface, de sorte qu'un changement donné dudit champ électrostatique soit opérationnel pour affecter ledit mécanisme de stockage et de libération de manière à effectuer la libération d'une substance stockée dans celui-ci.
